# EUROPEAN PATENT SPECIFICATION

(11) **EP 1 957 521 B1**
(45) Date of publication and mention of the grant of the patent: **19.02.2014**
(21) Application number: 06826205.4
(22) Date of filing: 17.10.2006
(51) Int. Cl.: C12Q 1/70

(54) **COMPOSITIONS FOR USE IN IDENTIFICATION OF INFLUENZA VIRUSES**
ZUSAMMENSETZUNGEN ZUR VERWENDUNG BEI DER IDENTIFIKATION VON INFLUENZA-VIREN
COMPOSITIONS DESTINEES A ETRE UTILISEES DANS L'IDENTIFICATION DE VIRUS DE LA GRIPPE

(30) Priority: 17.10.2005 US 728017 P
(43) Date of publication of application: 20.08.2008
(73) Proprietor: Ibis Biosciences, Inc., Carlsbad, CA 92008 (US)
(72) Inventor: SAMPATH, Rangarajan, San Diego, CA 92129 (US); HALL, Thomas, A., Oceanside, CA 92056 (US); ESHOO, Mark, W., Solana Beach, CA 92075 (US); LI, Feng, San Diego, CA 92121 (US)
(74) Representative: Hallybone, Huw George
(86) International application number: PCT/US2006/040747
(87) International publication number: WO 2007/047778

(56) References cited:
- WO-A-2005/012572
- SPACKMAN ERICA ET AL: "Development of a real-time reverse transcriptase PCR assay for type A influenza virus and the avian H5 and H7 hemagglutinin subtypes" JOURNAL OF CLINICAL MICROBIOLOGY, WASHINGTON, DC, US, vol. 40, no. 9, 1 September 2002 (2002-09-01), pages 3256-3260, XP002473651 ISSN: 0095-1137
- STONE BELINDA ET AL: "Rapid detection and simultaneous subtype differentiation of influenza A viruses by real time PCR" JOURNAL OF VIROLOGICAL METHODS, ELSEVIER BV, NL, vol. 117, no. 2, 1 May 2004 (2004-05-01), pages 103-112, XP002457249 ISSN: 0166-0934
- WARD C L ET AL: "Design and performance testing of quantitative real time PCR assays for influenza A and B viral load measurement" JOURNAL OF CLINICAL VIROLOGY, ELSEVIER, AMSTERDAM, NL, vol. 29, no. 3, 1 January 2004 (2004-01-01), pages 179-188, XP002457250 ISSN: 1386-6532
- DONOFRIO J C ET AL: "Detection of influenza A and B in respiratory secretions with the polymerase chain reaction" PCR METHODS & APPLICATIONS, COLD SPRING HARBOR LABORATORY PRESS, US, vol. 1, no. 4, 1 May 1992 (1992-05-01), pages 263-268, XP002457251 ISSN: 1054-9803

### SEQUENCE LISTING

<110> Isis Pharmaceuticals, Inc. Rangarajan sampath Thomas A. Hall Mark W. Eshoo Feng Li
<120> COMPOSITIONS FOR USE IN IDENTIFICATION OF INFLUENZA VIRUSES
<130> DIBIS-0080WO
<150> 60/728,017
   <151> 2005-10-17
<160> 136
<170> FastSEQ for Windows Version 4.0
<210> 1
   <211> 27
   <212> DNA
   <213> Artificial sequence
<220>
   <223> PCR Primer
<400> 1
   taccactgtg gaccatatgg ccataat 27
<210> 2
   <211> 31
   <212> DNA
   <213> Artificial sequence
<220>
   <223> PCR Primer
<400> 2
   tcggatattt cattgccatc atccacttca t 31
<210> 3
   <211> 28
   <212> DNA
   <213> Artificial Sequence
<220>
   <223> PCR Primer
<400> 3
   tcatggaggt tgttttccca aatgaagt 28
<210> 4
   <211> 26
   <212> DNA
   <213> Artificial Sequence
<220>
   <223> PCR Primer
<400> 4
   tctctctcca acatgtatgc caccat 26
<210> 5
   <211> 27
   <212> DNA
   <213> Artificial Sequence
<220>
   <223> PCR Primer
<400> 5
   tcccattgta ctggcataca tgcttga 27
<210> 6
   <211> 27
   <212> DNA
   <213> Artificial sequence
<220>
   <223> PCR Primer
<400> 6
   tatgaactca gctgatgttg ctcctgc 27
<210> 7
   <211> 27
   <212> DNA
   <213> Artificial sequence
<220>
   <223> PCR Primer
<400> 7
   tctcaccaag gaaatgcctc cagatga 27
<210> 8
   <211> 31
   <212> DNA
   <213> Artificial sequence
<220>
   <223> PCR Primer
<400> 8
   tccaagtaga ttctcttggt attcctgctt c 31
<210> 9
   <211> 28
   <212> DNA
   <213> Artificial Sequence
<220>
   <223> PCR Primer
<400> 9
   tggctaacaa gagaatgctg gaagaagc 28
<210> 10
   <211> 31
   <212> DNA
   <213> Artificial Sequence
<220>
   <223> PCR Primer
<400> 10
   tatacaagag gcgcttgcaa gaacatgatc c 31
<210> 11
   <211> 27
   <212> DNA
   <213> Artificial Sequence
<220>
   <223> PCR Primer
<400> 11
   tgctaaggca gctcaaatga tgacagt 27
<210> 12
   <211> 26
   <212> DNA
   <213> Artificial sequence
<220>
   <223> PCR Primer
<400> 12
   tctgctcatt gcccatctca ttctta 26
<210> 13
   <211> 22
   <212> DNA
   <213> Artificial Sequence
<220>
   <223> PCR Primer
<400> 13
   tggcgtctca aggcaccaaa cg 22
<210> 14
   <211> 24
   <212> DNA
   <213> Artificial sequence
<220>
   <223> PCR Primer
<400> 14
   tctgatctca gtggcattct ggcg 24
<210> 15
   <211> 31
   <212> DNA
   <213> Artificial Sequence
<220>
   <223> PCR Primer
<400> 15
   tacatccaga tgtgcactga actcaaactc a 31
<210> 16
   <211> 31
   <212> DNA
   <213> Artificial Sequence
<220>
   <223> PCR Primer
<400> 16
   tcgtcaaatg cagagagcac cattctctct a 31
<210> 17
   <211> 20
   <212> DNA
   <213> Artificial sequence
<220>
   <223> PCR Primer
<400> 17
   tggcgccaag cgaacaatgg 20
<210> 18
   <211> 31
   <212> DNA
   <213> Artificial Sequence
<220>
   <223> PCR Primer
<400> 18
   tggcatcatt cagattggaa tgccagatca t 31
<210> 19
   <211> 22
   <212> DNA
   <213> Artificial sequence
<220>
   <223> PCR Primer
<400> 19
   tggcatgcca ttctgcagca tt 22
<210> 20
   <211> 32
   <212> DNA
   <213> Artificial sequence
<220>
   <223> PCR Primer
<400> 20
   ttctcatttg aagcaatttg aactcctcta gt 32
<210> 21
   <211> 30
   <212> DNA
   <213> Artificial sequence
<220>
   <223> PCR Primer
<400> 21
   taccagagga gttcaaattg cttcaaatga 30
<210> 22
   <211> 24
   <212> DNA
   <213> Artificial Sequence
<220>
   <223> PCR Primer
<400> 22
   tccactcctg gtccttatag ccca 24
<210> 23
   <211> 22
   <212> DNA
   <213> Artificial sequence
<220>
   <223> PCR Primer
<400> 23
   tgagtcttcg agctctcgga cg 22
<210> 24
   <211> 25
   <212> DNA
   <213> Artificial sequence
<220>
   <223> PCR Primer
<400> 24
   tcatgtcaaa ggaaggcacg atcgg 25
<210> 25
   <211> 22
   <212> DNA
   <213> Artificial Sequence
<220>
   <223> PCR Primer
<400> 25
   tccagatgag caacgcaaag cc 22
<210> 26
   <211> 29
   <212> DNA
   <213> Artificial Sequence
<220>
   <223> PCR Primer
<400> 26
   tccacttcct tacaaatggc aatgtaggc 29
<210> 27
   <211> 31
   <212> DNA
   <213> Artificial sequence
<220>
   <223> PCR Primer
<400> 27
   tagtattgat gatggcatgc tttggacttg c 31
<210> 28
   <211> 30
   <212> DNA
   <213> Artificial Sequence
<220>
   <223> PCR Primer
<400> 28
   tcattctgtt tctcaactta agagggtggc 30
<210> 29
   <211> 28
   <212> DNA
   <213> Artificial Sequence
<220>
   <223> PCR Primer
<400> 29
   ttcatgtctt gcttcggagc tgcctatg 28
<210> 30
   <211> 26
   <212> DNA
   <213> Artificial Sequence
<220>
   <223> PCR Primer
<400> 30
   tgttcctttg ctggaacatg gaaacc 26
<210> 31
   <211> 27
   <212> DNA
   <213> Artificial Sequence
<220>
   <223> PCR Primer
<400> 31
   tccaatcatc agaccagcaa cccttgc 27
<210> 32
   <211> 26
   <212> DNA
   <213> Artificial sequence
<220>
   <223> PCR Primer
<400> 32
   tccgatatca gcttcactgc ttgtgg 26
<210> 33
   <211> 28
   <212> DNA
   <213> Artificial sequence
<220>
   <223> PCR Primer
<400> 33
   tctacaacca gatgatggtc aaagctgg 28
<210> 34
   <211> 27
   <212> DNA
   <213> Artificial sequence
<220>
   <223> PCR Primer
<400> 34
   tgcagccaat agaattcttt ccacagc 27
<210> 35
   <211> 28
   <212> DNA
   <213> Artificial sequence
<220>
   <223> PCR Primer
<400> 35
   tgacaagacc aatcctgtca cctctgac 28
<210> 36
   <211> 24
   <212> DNA
   <213> Artificial sequence
<220>
   <223> PCR Primer
<400> 36
   ttggacaaag cgtctacgct gcag 24
<210> 37
   <211> 26
   <212> DNA
   <213> Artificial sequence
<220>
   <223> PCR Primer
<400> 37
   tgagtcttct aaccgaggtc gaaacg 26
<210> 38
   <211> 23
   <212> DNA
   <213> Artificial sequence
<220>
   <223> PCR Primer
<400> 38
   tctgcgcgat ctcggctttg agg 23
<210> 39
   <211> 28
   <212> DNA
   <213> Artificial Sequence
<220>
   <223> PCR Primer
<400> 39
   tcttgccagt tgtatgggcc tcatatac 28
<210> 40
   <211> 23
   <212> DNA
   <213> Artificial sequence
<220>
   <223> PCR Primer
<400> 40
   tgggagtcag caatctgctc aca 23
<210> 41
   <211> 26
   <212> DNA
   <213> Artificial sequence
<220>
   <223> PCR Primer
<400> 41
   tgtcgctgtt tggagacaca attgcc 26
<210> 42
   <211> 26
   <212> DNA
   <213> Artificial Sequence
<220>
   <223> PCR Primer
<400> 42
   tccattccaa ggcagagtct aggtca 26
<210> 43
   <211> 26
   <212> DNA
   <213> Artificial Sequence
<220>
   <223> PCR Primer
<400> 43
   tcatcacaga gcccctatca ggaatg 26
<210> 44
   <211> 30
   <212> DNA
   <213> Artificial Sequence
<220>
   <223> PCR Primer
<400> 44
   tggccttctg ctatttcaaa tgcttcatga 30
<210> 45
   <211> 25
   <212> DNA
   <213> Artificial Sequence
<220>
   <223> PCR Primer
<400> 45
   tctgtgcttt gtgcgagaaa caagc 25
<210> 46
   <211> 26
   <212> DNA
   <213> Artificial Sequence
<220>
   <223> PCR Primer
<400> 46
   tgtgttcata gctgagacca tctgca 26
<210> 47
   <211> 30
   <212> DNA
   <213> Artificial sequence
<220>
   <223> PCR Primer
<400> 47
   tggagacttc ttgggagtgg agtcaatgat 30
<210> 48
   <211> 27
   <212> DNA
   <213> Artificial Sequence
<220>
   <223> PCR Primer
<400> 48
   tcggatgtct ggtgtgtagt cgtctgg 27
<210> 49
   <211> 27
   <212> DNA
   <213> Artificial sequence
<220>
   <223> PCR Primer
<400> 49
   tgagaccagg acagcaataa tttcagc 27
<210> 50
   <211> 25
   <212> DNA
   <213> Artificial sequence
<220>
   <223> PCR Primer
<400> 50
   tgcattgtgg tggcttctcc agaca 25
<210> 51
   <211> 27
   <212> DNA
   <213> Artificial Sequence
<220>
   <223> PCR Primer
<400> 51
   taatgtctca gaaggtggaa gaacagc 27
<210> 52
   <211> 25
   <212> DNA
   <213> Artificial sequence
<220>
   <223> PCR Primer
<400> 52
   tctccaaggc cagtaatacc agcaa 25
<210> 53
   <211> 29
   <212> DNA
   <213> Artificial sequence
<220>
   <223> PCR Primer
<400> 53
   tttgtgcgac aatgcttcaa tccgatgat 29
<210> 54
   <211> 30
   <212> DNA
   <213> Artificial Sequence
<220>
   <223> PCR Primer
<400> 54
   tgtgcatatt gcagcaaatt tgtttgtttc 30
<210> 55
   <211> 23
   <212> DNA
   <213> Artificial Sequence
<220>
   <223> PCR Primer
<400> 55
   tgggattcct ttcgtcagtc cga 23
<210> 56
   <211> 27
   <212> DNA
   <213> Artificial Sequence
<220>
   <223> PCR Primer
<400> 56
   tggagaagtt cggtgggaga ctttggt 27
<210> 57
<220>
<400> 57
   000
<210> 58
<220>
<400> 58
   000
<210> 59
   <211> 23
   <212> DNA
   <213> Artificial Sequence
<220>
   <223> PCR Primer
<400> 59
   tcgtcagtcc gagagaggcg aag 23
<210> 60
   <211> 30
   <212> DNA
   <213> Artificial Sequence
<220>
   <223> PCR Primer
<400> 60
   ttcggttcga atccatccac ataggctcta 30
<210> 61
   <211> 24
   <212> DNA
   <213> Artificial Sequence
<220>
   <223> PCR Primer
<400> 61
   tcttagggac aacctggaac ctgg 24
<210> 62
   <211> 28
   <212> DNA
   <213> Artificial Sequence
<220>
   <223> PCR Primer
<400> 62
   taacccaggg atcattaatc aggcactc 28
<210> 63
   <211> 32
   <212> DNA
   <213> Artificial Sequence
<220>
   <223> PCR Primer
<400> 63
   tcacaatggc agaatttagt gaagatcctg aa 32
<210> 64
   <211> 35
   <212> DNA
   <213> Artificial sequence
<220>
   <223> PCR Primer
<400> 64
   tcttgtcctt ctaatgctgt atatgctttt ccttc 35
<210> 65
   <211> 32
   <212> DNA
   <213> Artificial Sequence
<220>
   <223> PCR Primer
<400> 65
   tctgttccag ctggtttctc caattttgaa gg 32
<210> 66
   <211> 30
   <212> DNA
   <213> Artificial Sequence
<220>
   <223> PCR Primer
<400> 66
   tgactgatac taagggagac atccttgcta 30
<210> 67
   <211> 33
   <212> DNA
   <213> Artificial Sequence
<220>
   <223> PCR Primer
<400> 67
   tgagctacca gaagttccat ataatgcctt tct 33
<210> 68
   <211> 36
   <212> DNA
   <213> Artificial Sequence
<220>
   <223> PCR Primer
<400> 68
   tagtgttgag tacttttcta gacattcttt ggctaa 36
<210> 69
   <211> 33
   <212> DNA
   <213> Artificial sequence
<220>
   <223> PCR Primer
<400> 69
   tggaagttgt ggagggactg tgtaaataca ata 33
<210> 70
   <211> 26
   <212> DNA
   <213> Artificial Sequence
<220>
   <223> PCR Primer
<400> 70
   tcctgtggcc cacttggcat aattgg 26
<210> 71
   <211> 33
   <212> DNA
   <213> Artificial sequence
<220>
   <223> PCR Primer
<400> 71
   tgtgatgagt atttgagtac aaatgggagt gat 33
<210> 72
   <211> 30
   <212> DNA
   <213> Artificial Sequence
<220>
   <223> PCR Primer
<400> 72
   tagctcattt tgtaaagaca ctgcagttcc 30
<210> 73
   <211> 28
   <212> DNA
   <213> Artificial Sequence
<220>
   <223> PCR Primer
<400> 73
   tcttgcaact gctgctgatt ttcttagg 28
<210> 74
   <211> 31
   <212> DNA
   <213> Artificial Sequence
<220>
   <223> PCR Primer
<400> 74
   tcatttcaat gatggtttct tcattgtctg g 31
<210> 75
   <211> 32
   <212> DNA
   <213> Artificial Sequence
<220>
   <223> PCR Primer
<400> 75
   tgtctgccag ttggtgggaa tgagaagaag gc 32
<210> 76
   <211> 26
   <212> DNA
   <213> Artificial sequence
<220>
   <223> PCR Primer
<400> 76
   tcattccatt tagtattgtc tccagt 26
<210> 77
<220>
<400> 77
   000
<210> 78
   <211> 26
   <212> DNA
   <213> Artificial Sequence
<220>
   <223> PCR Primer
<400> 78
   tcatcagaag attggagccc atccca 26
<210> 79
   <211> 26
   <212> DNA
   <213> Artificial sequence
<220>
   <223> PCR Primer
<400> 79
   tgtcgtggaa tgatgatggg catgtt 26
<210> 80
   <211> 27
   <212> DNA
   <213> Artificial Sequence
<220>
   <223> PCR Primer
<400> 80
   ttcatcagaa gattggagcc catccca 27
<210> 81
   <211> 28
   <212> DNA
   <213> Artificial Sequence
<220>
   <223> PCR Primer
<400> 81
   tggaatgatg atgggcatgt tcaatatg 28
<210> 82
   <211> 32
   <212> DNA
   <213> Artificial sequence
<220>
   <223> PCR Primer
<400> 82
   tcaaaatcat cagaagattg gagcccatcc ca 32
<210> 83
   <211> 24
   <212> DNA
   <213> Artificial sequence
<220>
   <223> PCR Primer
<220>
   <221> misc_feature
   <222> 19
   <223> n = I
<400> 83
   tcctggaatg atgatgggna tgtt 24
<210> 84
   <211> 25
   <212> DNA
   <213> Artificial Sequence
<220>
   <223> PCR Primer
<400> 84
   tcatcagaag attggagccc atccc 25
<210> 85
<220>
<400> 85
   000
<210> 86
   <211> 25
   <212> DNA
   <213> Artificial sequence
<220>
   <223> PCR Primer
<220>
   <221> misc_feature
   <222> 18
   <223> n = I
<400> 86
   tcatcagaag attggagncc atccc 25
<210> 87
<220>
<400> 87
   000
<210> 88
   <211> 25
   <212> DNA
   <213> Artificial Sequence
<220>
   <223> PCR Primer
<220>
   <221> misc_feature
   <222> 15, 18
   <223> n = I
<400> 88
   tcatcagaag attgnagncc atccc 25
<210> 89
<220>
<400> 89
   000
<210> 90
   <211> 25
   <212> DNA
   <213> Artificial sequence
<220>
   <223> PCR Primer
<220>
   <221> misc_feature
   <222> 9, 15, 18, 21
   <223> n = I
<400> 90
   tcatcagang attgnagncc ntccc 25
<210> 91
<220>
<400> 91
   000
<210> 92
   <211> 25
   <212> DNA
   <213> Artificial sequence
<220>
   <223> PCR Primer
<220>
   <221> misc_feature
   <222> 9, 15, 18
   <223> n = I
<400> 92
   tcatcagang attgnagncc atccc 25
<210> 93
   <211> 26
   <212> DNA
   <213> Artificial Sequence
<220>
   <223> PCR Primer
<220>
   <221> misc_feature
   <222> 21
   <223> n = I
<400> 93
   tgtcctggaa tgatgatggg natgtt 26
<210> 94
<220>
<400> 94
   000
<210> 95
<220>
<400> 95
   000
<210> 96
<220>
<400> 96
   000
<210> 97
<220>
<400> 97
   000
<210> 98
<220>
<400> 98
   000
<210> 99
   <211> 26
   <212>,DNA
   <213> Artificial Sequence
<220>
   <223> PCR Primer
<220>
   <221> misc_feature
   <222> 3, 9, 21
   <223> n = I
<400> 99
   tgncctggna tgatgatggg natgtt 26
<210> 100
<220>
<400> 100
   000
<210> 101
<220>
<400> 101
   000
<210> 102
<220>
<400> 102
   000
<210> 103
   <211> 26
   <212> DNA
   <213> Artificial Sequence
<220>
   <223> PCR Primer
<220>
   <221> misc_feature
   <222> 3, 6, 9, 21
   <223> n = I
<400> 103
   tgnccnggna tgatgatggg natgtt 26
<210> 104
<220>
<400> 104
   000
<210> 105
<220>
<400> 105
   000
<210> 106
<220>
<400> 106
   000
<210> 107
<220>
<400> 107
   000
<210> 108
<220>
<400> 108
   000
<210> 109
<220>
<400> 109
   000
<210> 110
<220>
<400> 110
   000
<210> 111
<220>
<400> 111
   000
<210> 112
<220>
<400> 112
   000
<210> 113
   <211> 26
   <212> DNA
   <213> Artificial Sequence
<220>
   <223> PCR Primer
<220>
   <221> misc_feature
   <222> 4, 5, 6, 13, 15, 21
   <223> n = I i
<400> 113
   tgtnnnggaa tgntnatggg natgtt 26
<210> 114
   <211> 25
   <212> DNA
   <213> Artificial sequence
<220>
   <223> PCR Primer
<220>
   <221> misc_feature
   <222> 9, 15, 18, 22, 23
   <223> n = I
<400> 114
   tcatcagang attgnagncc anncc 25
<210> 115
   <211> 23
   <212> DNA
   <213> Artificial sequence
<220>
   <223> PCR Primer
<400> 115
   tatcagaaac ggatgggggt gca 23
<210> 116
   <211> 28
   <212> DNA
   <213> Artificial sequence
<220>
   <223> PCR Primer
<400> 116
   tgatcaagaa tccacaatat caagtgca 28
<210> 117
   <211> 25
   <212> DNA
   <213> Artificial Sequence
<220>
   <223> PCR Primer
<400> 117
   tgagtcttct aaccgaggtc gaaac 25
<210> 118
   <211> 27
   <212> DNA
   <213> Artificial Sequence
<220>
   <223> PCR Primer
<400> 118
   tccacaatat caagtgcaag atcccaa 27
<210> 119
   <211> 34
   <212> DNA
   <213> Artificial Sequence
<220>
   <223> PCR Primer
<400> 119
   tccaggacat actgatgagg atgtcaaaaa tgca 34
<210> 120
   <211> 24
   <212> DNA
   <213> Artificial Sequence
<220>
   <223> PCR Primer
<400> 120
   tgcttcccca agcgaatctc tgta 24
<210> 121
<220>
<400> 121
   000
<210> 122
<220>
<400> 122
   000
<210> 123
   <211> 28
   <212> DNA
   <213> Artificial Sequence
<220>
   <223> PCR Primer
<400> 123
   tgtcaaaaat gcaattgggg tcctcatc 28
<210> 124
   <211> 31
   <212> DNA
   <213> Artificial Sequence
<220>
   <223> PCR Primer
<400> 124
   tcattactgc ttctccaagc gaatctctgt a 31
<210> 125
   <211> 26
   <212> DNA
   <213> Artificial sequence
<220>
   <223> PCR Primer
<400> 125
   tgtcctggaa tgatgatggg catgtt 26
<210> 126
   <211> 25
   <212> DNA
   <213> Artificial Sequence
<220>
   <223> PCR Primer
<400> 126
   tcatcagagg attggagtcc atccc 25
<210> 127
   <211> 26
   <212> DNA
   <213> Artificial Sequence
<220>
   <223> PCR Primer
<400> 127
   tgtcctggaa tgatgattgg catgtt 26
<210> 128
<220>
<400> 128
   000
<210> 129
<220>
<400> 129
   000
<210> 130
   <211> 25
   <212> DNA
   <213> Artificial sequence
<220>
   <223> PCR Primer
<400> 130
   tcgtcagagg attggagtcc atccc 25
<210> 131
   <211> 26
   <212> DNA
   <213> Artificial sequence
<220>
   <223> PCR Primer
<220>
   <221> misc_feature
   <222> 21
   <223> n = I
<400> 131
   tgtcctggaa tgatgattgg natgtt 26
<210> 132
   <211> 25
   <212> DNA
   <213> Artificial Sequence
<220>
   <223> PCR Primer
<220>
   <221> misc_feature
   <222> 15
   <223> n = I
<400> 132
   tcgtcagagg attgnagtcc atccc 25
<210> 133
   <211> 28
   <212> DNA
   <213> Artificial Sequence
<220>
   <223> PCR Primer
<220>
   <221> misc_feature
   <222> 16
   <223> n = I
<400> 133
   tggaatgatg attggnatgt tcaacatg 28
<210> 134
   <211> 31
   <212> DNA
   <213> Artificial Sequence
<220>
   <223> PCR Primer
<220>
   <221> misc_feature
   <222> 21 -
   <223> n = I
<400> 134
   tcaaaatcgt cagaggattg nagtccatcc c 31
<210> 135
   <211> 25
   <212> DNA
   <213> Artificial Sequence
<220>
   <223> PCR Primer
<400> 135
   tgccagttgg tggtaatgag aagaa 25
<210> 136
   <211> 32
   <212> DNA
   <213> Artificial sequence
<220>
   <223> PCR Primer
<400> 136
   tgacattcat tccatttggt gttgtctcca gt 32

## Description

### FIELD OF THE INVENTION

The present invention relates generally to the field of genetic identification and quantification of influenza viruses and provides methods, compositions and kits useful for this purpose, as well as others, when combined with molecular mass analysis.

### BACKGROUND OF THE INVENTION

Influenza virus belongs to the *orthomyxovirdae* family, which consists of influenza A, B, C and thogotovirus. It is an enveloped RNA virus. The envelope is primarily a matrix protein (MP) and two glycoproteins called nuraminidase (NA) and hemagglutinin (HA). NA and HA are present on the surface and play important roles in infecting a host cell. Inside the envelope are segmented single stranded RNA and nucleoprotein (NP). The function of NP is to encapsulate RNA and to play a role in transcription, replication and packaging. The classification of influenza typing (A, B or C) is based on the different antigenicity of NP and MP. Influenza A is further categorized into sub-types based on serologic cross reactivity of HA or NA antibodies. Only one sub-type of HA and one sub-type of NA is known for influenza B. The current subtypes of influenza A viruses found in people are A(H1N1) and A(H3N2). A total of 15 different HA types have been described and 9 different NA types, although not all combinations of these segments are known to be present (Armano, Y et al., Anal Bioanal Chem (2005) 381: 156-164)

Influenza types A or B viruses cause epidemics of disease almost every winter. Influenza A viruses are found in many different animals, including ducks, chickens, pigs, whales, horses, and seals. Influenza B viruses circulate widely only among humans. Influenza type C infections cause a mild respiratory illness and are not thought to cause epidemics.

Many of these types are specific to single host species and do not jump species. However, avian H5N1 with episodic transmissions to humans, as well as the recently described canine/equine H3N8 strains, are clearly adapted to multiple species and pose a distinct potential for a pandemic. Similarly, pigs can be infected with both human and avian influenza viruses in addition to swine influenza viruses. Thus, detection of influenza A viruses with the corresponding HA and NA types is clearly necessary to track outbreak of novel pandemic strains.

Influenza viruses change in two different ways. One is called "antigenic drift." These are small and gradual changes to the virus' HA and NA proteins that happen continually over time. Antigenic drift produces new virus strains that may not be recognized by the body's immune system. The other type of change is called "antigenic shift." Antigenic shift is an abrupt, major change in the influenza A viruses, resulting in new HA and/or new HA and NA proteins in influenza viruses that infect humans. Shift results in a new influenza A subtype. When a shift happens, most people have little or no protection against the new virus. A pandemic is possible when an influenza A virus makes an antigenic shift and acquires a new HA or HA+NA. This shift results in a new or "novel" virus to which the general population has no immunity. The appearance of a novel virus is the first step toward a pandemic. However, the novel influenza A virus also must spread easily from person to person (and cause serious disease) for a pandemic to occur. While influenza viruses are changing by antigenic drift all the time, antigenic shift happens only occasionally. Type A viruses undergo both kinds of changes; influenza type B viruses change only by the more gradual process of antigenic drift.

Conventional virologic methods for influenza virus analysis are well established. Viral isolation culture with immunologic confirmation of viral antigen is the current "gold standard" for virus detection. The most common cell line for influenza culture is the Madin-Darby Canine Kidney cell (MDCK) because the MDCK cell line supports the growth of influenza A, B and C. Following a 2-10 day viral culture the virus is detected using an immunoassay procedure such as immunofluorescence or ELISA followed by a serologic or molecular biologic assay for virus characterization. Viral detection methods can include complement fixation, hemagglutinin-inhibition, and PCR. These detection and characterization methods provide yes/no answers to the question of whether a known influenza type or sub-type is present in a mixture. (Amano, Y et al., Anal Bioanal Chem (2005) 381: 156-164). Unfortunately, these methods for detecting and characterizing influenza are only capable of identifying types and sub-types that are already known. They are not effective for is providing information about an influenza virus with an unknown type or sub-type.

In order to deliver an effective antiviral treatment, timely diagnosis is necessary. Effective therapy must be delivered within 48 hours of symptom onset, which is far shorter than even the viral culture methods currently used. Conventional detection and characterization methods fail when the virus is novel due to an antigenic shift or drift that renders it undetectable or uncharacterizable. (Amano, Y et al., Anal Bioanal Chem (2005) 381:156-164; Manalito, M.J., American Family Physician, (2003) 67:111-118; Li, J et al., J. Clin. Microbiol. (2001) 39: 696-704).

Microarrays have been used to provide a more rapid screening method for the detection of influenza virus. United States Patent No.: 6,852,487 issued to Barany *et al.* and assigned to Cornell Research Foundation, Inc. describe a microarray for detecting nucleic acid differences. This patent describes compositions and methods for detecting one or more differing nucleic acid sequences. Nucleic acid regions suspected of having a nucleotide mutation, polymorphism, deletion or insertion, are PCR amplified. The amplified nucleic acid is then used as a template in a ligase detection reaction. In this assay, two primers are designed to hybridize on adjacent sides of an area suspected of having the mutation. The primers hybridize with the amplified product in the presence of a ligase and if the primers have full complementarity with the template then the primers are ligated. The primers are then hybridized with a probe that is covalently attached to a microarray and is assayed to determine whether the primers ligated. This assay requires prior knowledge mutation's location so that the primers can be designed to hybridize on adjacent sides. Thus, this assay is not able to detect previously unknown mutations.

WO 2005/012572 discloses a screening method of reassortant influenza viruses using primer dependent multiplex RT-PCR.

There is a need in the art for an assay that will rapidly detect and characterize influenza virus. This need includes that the assay should specifically detect and characterize both known and unknown viruses. Detection and characterization of unknown viruses should include those harboring any mutation and without the need for additional detection/characterization assays. Rapid detection and characterization will allow for timely introduction of a proper antiviral therapy, and moreover, will allow for control of influenza epidemics by rapidly identifying new sub-types.

### SUMMARY OF THE INVENTION

The invention provides an oligonucleotide primer pair wherein each primer member of the primer pair is independently up to 35 nucleobases in length and wherein the forward primer comprises SEQ ID NO: 125 and the reverse primer comprises SEQ ID NO: 126.

The invention also provides a kit comprising the oligonucleotide primer pair of the invention.

The invention further provides a method for identification of an influenza virus comprising: amplifying, in a sample suspected of comprising at least one bioagent, nucleic acid from said influenza virus using the composition of the invention to obtain an amplicon; determining the molecular mass of said amplicon using a mass spectrometer; calculating the base composition of said amplicon from said molecular mass; and comparing said molecular mass or base composition with a plurality of molecular masses or base compositions indexed to known influenza virus bioagents and primer pairs, wherein a match between said molecular mass or base composition and a member of said plurality of molecular masses or base compositions identifies said unknown influenza virus.

The invention also provides a method of determining the presence or absence of an influenza virus in a sample comprising: amplifying, in a sample suspected of comprising at least one bioagent, nucleic acid from said sample using the composition of the invention to obtain an amplicon; determining the molecular mass of said amplicon using a mass spectrometer; calculating the base composition of said amplicon from said molecular mass; and comparing said molecular mass or base composition of said amplicon with a collection of molecular masses or base compositions indexed to a known bioagent and to a primer pair, wherein a match between the measured molecular mass or calculated base composition and one of the molecular mass or base composition in the collection indicates the presence of influenza virus in said sample.

The invention further provides combination of at least three primer pairs for identifying the genus, species, subtype and genotype of at least two bioagents belonging to the orthomyxovirdae family, wherein the primer pairs individually bind to one of a gene sequence comprising PB1, NUC, M1, PA, NS1, NS2, and PB2 and thereby generate corresponding amplicons from each of the two or more bioagents, the amplicons being compatible with a mass spectrometer for generating a molecular mass measurement and for calculating a base composition signature that corresponds to each of the two or more bioagents, and wherein the combination comprises a primer pair comprising a forward primer that has 100% sequence identity with SEQ ID NO: 125, and a reverse primer that has 100% sequence identity with SEQ ID NO: 126.

The invention further provides a method for generating a base composition signature to identify at least one member of the orthomyxovirdae family comprising the steps of: (a) obtaining a primer pair according to the invention; (b) contacting the primer pair to a sample suspected of comprising a unknown bioagent; (c) generating an amplicon; (d) obtaining a mass measurement of the amplicon using a mass spectrometer; and (e) calculating a base composition signature from the molecular mass data, wherein the base composition signature is queried against a database of base composition signatures indexed to the corresponding primer pairs and the bioagent identity.

### SUMMARY OF THE DISCLOSURE

Provided herein are, *inter alia,* methods of identifying members of the *orthomyxovirdae* family. Preferably, the genus of the members is identified, more preferably the species of the members is identified, more preferably still the sub-species of the members is identified, more preferably the strain of the members is identifies, and most preferably the genotype of the members is identified. Also provided are oligonucleotide primers, compositions and kits containing the oligonucleotide primers, which define viral bioagent identifying amplicons and, upon amplification, produce amplicons whose molecular masses provide the means to identify influenza viruses at the sub-species level.

Provided herein are primers and compositions comprising pairs of primers; kits containing the same; and methods for their use in identification of influenza viruses. The primers are designed to produce viral bioagent identifying nucleic acid amplicons. The amplicons are preferably generated from sections of nucleic acid encoding genes essential to virus replication. Compositions comprising pairs of primers and the kits containing the same are designed to provide species and sub-species characterization of influenza viruses.

In some embodiments, methods for identification of influenza viruses are provided. Nucleic acid from the influenza virus is amplified using the primers described above to obtain an amplicon. The molecular mass of this amplicon is measured using mass spectrometry. A base composition of the amplicon is calculated from the molecular mass. The molecular mass or base composition is compared with a plurality of molecular masses or base compositions of known influenza virus identifying amplicons, wherein a match between the molecular mass or base composition and a member of the plurality of molecular masses or base compositions identifies the influenza virus.

In some embodiments, methods of detecting the presence or absence of an influenza virus in a sample are provided. Nucleic acid from the sample is amplified using the composition described above to obtain an amplicon The molecular mass of this amplicon is determined. A base composition of the amplicon is determined from the molecular mass. The molecular mass or base composition of the amplicon is compared with known molecular masses or base compositions of one or more known influenza virus identifying amplicons, wherein a match between the molecular mass or base composition of the amplicon and the molecular mass or base composition of one or more known influenza virus identifying amplicons indicates the presence of the influenza virus in the sample.

In some embodiments, methods for determination of the quantity of an unknown influenza virus in a sample are provided. The sample is contacted with the composition described above and a known quantity of a calibration polynucleotide comprising a calibration sequence. Nucleic acid from the unknown influenza virus in the sample is concurrently amplified with the composition described above and nucleic acid from the calibration polynucleotide in the sample is concurrently amplified with the composition described above to obtain a first amplicon comprising an influenza virus identifying amplicon and a second amplicon comprising a calibration amplicon. The molecular mass and abundance for the influenza virus identifying amplicon and the calibration amplicon is determined. The influenza virus identifying amplicon is distinguished from the calibration amplicon based on molecular mass, wherein comparison of influenza virus identifying amplicon abundance and calibration amplicon abundance indicates the quantity of influenza virus in the sample. The base composition of the influenza virus identifying amplicon is determined.

### BRIEF DESCRIPTION OF THE DRAWINGS

The foregoing summary and detailed description is better understood when read in conjunction with the accompanying drawings which are included by way of example and not by way of limitation.

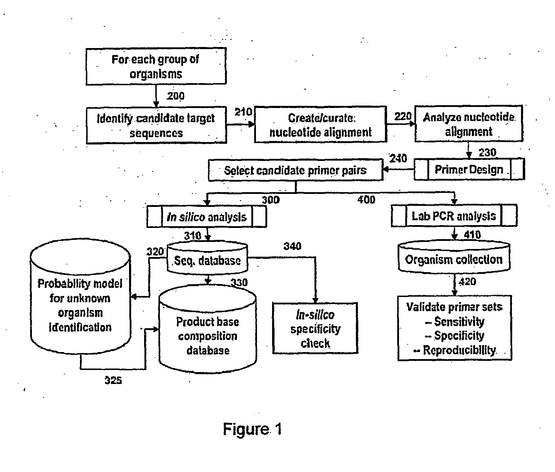

**Figure 1** is a process diagram illustrating a representative primer selection process.

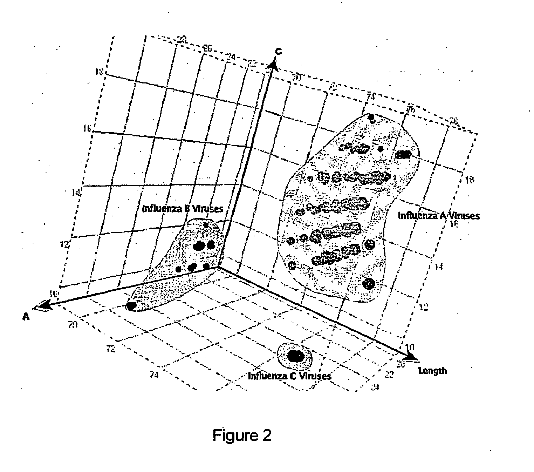

**Figure 2** is a representative three dimensional plot of base compositions of influenza viruses showing length, A and C counts of amplicons obtained with primer pair no: 1299 (SEQ ID NOs: 81:82). Each sphere represents one or more viral isolates and is based on all available nucleotide sequences for influenza viruses in GenBank.

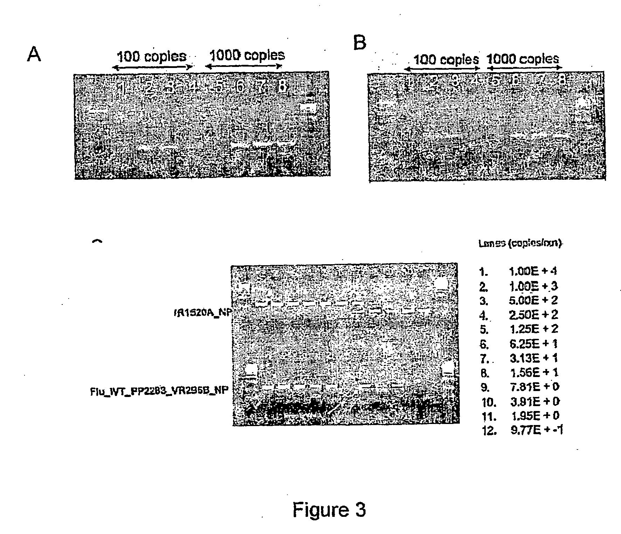

**Figure 3** provides validation data for influenza primers tested against *in vitro* transcribed cDNA. Primers targeted to PB1 (panels A and B) and NUC (or NP) (panel C) were tested. Lane assignments for the top panel are as follows: L1/L5: Water; L2-L4 and L6-8 were three of the broad PB1 primers. Primers in lanes 2 and 3 (1297 and 1298) were sensitive to ∼100 copies of the input material for both influenza A (panel A) and influenza B (panel B). Panel C shows two different primers, VIR1268 (influenza A) and VIR1274 (influenza B) that are specific to either influenza A or B. These primers were sensitive to 3-15 copies of input template.

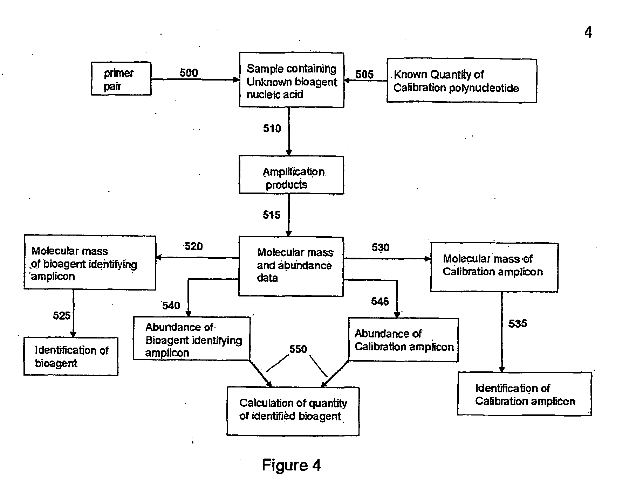

**Figure 4** is a process diagram illustrating an embodiment of the calibration method.

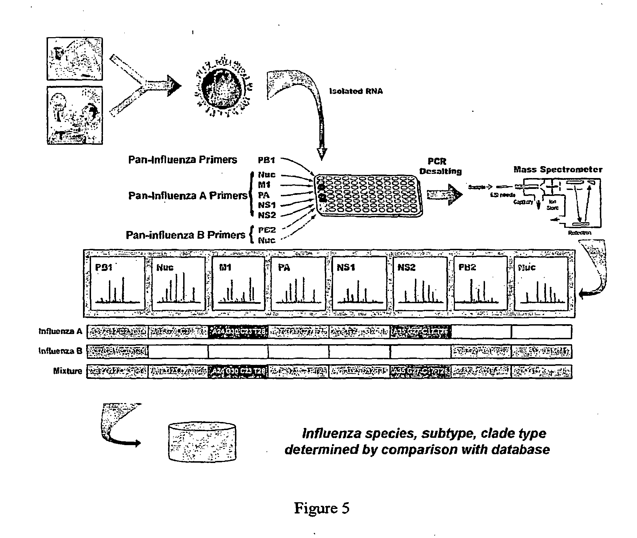

**Figure 5****:** Is a representative of one influenza virus surveillance schema. A panel of primers that includes a pan-influenza virus primer (PB 1) and additional influenza A-specific and influenza B-specific primers detect all known influenza viruses from different hosts (e.g., avian, human, swine). ESI/MS analysis of PCR amplicons provides the base-composition signatures from the input sample. The signature is compared to a database of known base compositions for identification and typing.

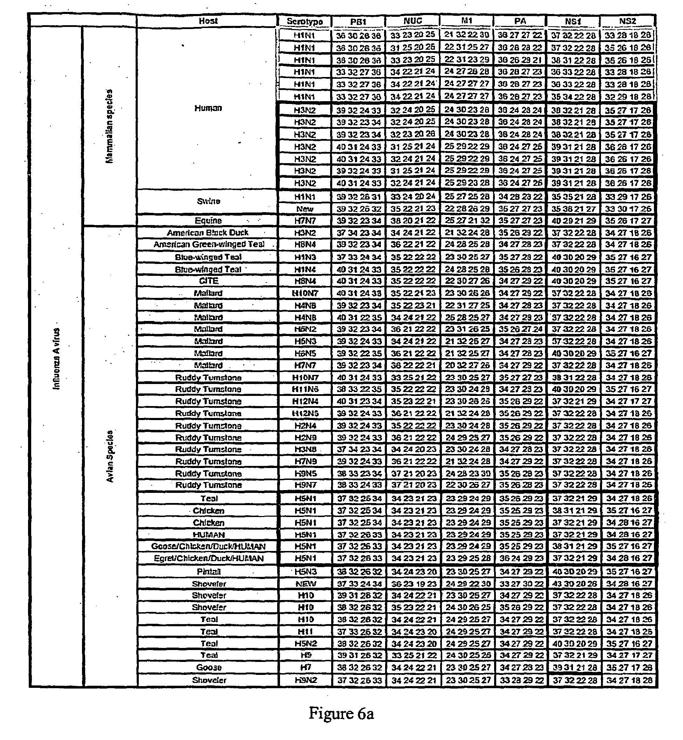

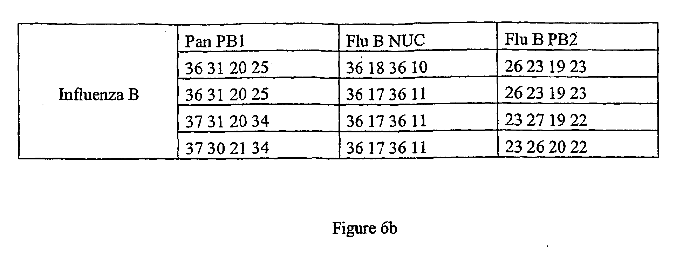

**Figures 6a** **and** **6b****.** Influenza virus target genes and observed base composition signatures for a diverse panel of viral isolates.

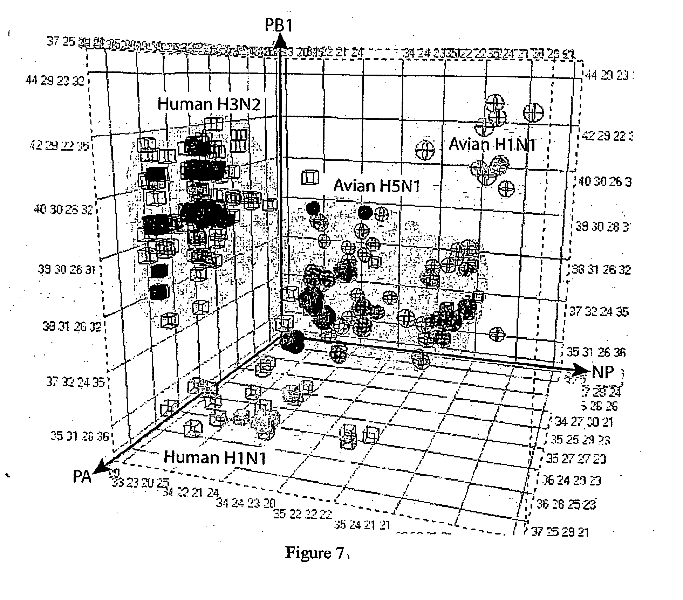

**Figure 7****.** Detection and characterization of important human and avian influenza virus sub-types. Each axis represents base composition signatures from a single primer pair. Open symbols are calculated base compositions determined from published sequences and solid symbols represent measurements in the below examples.

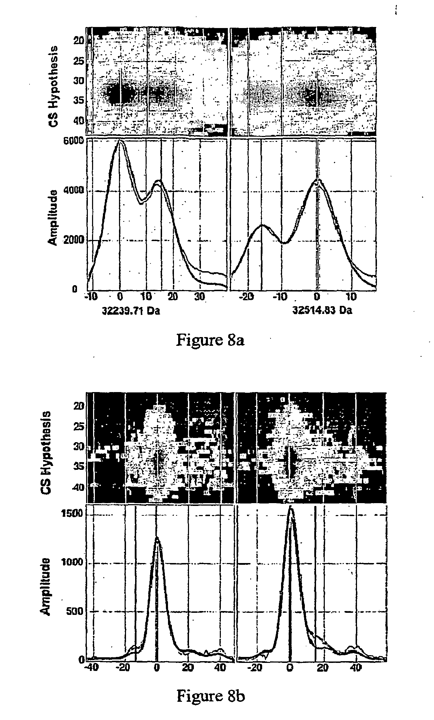

**Figures 8a to 8d** are heat maps and spectral plots indicating the detection of mixed viral populations. The heat maps are a charge state representation of the data; the spectral plots are created by filtering the charge state response to create the signal representations vs. mass. The main peaks on the spectral plots are the primary amplicons and appear as a hot spot in the upper heat map images. The secondary amplicon appears in the heat map as a "cloudy" region to the left and right for the reverse and forward strands, respectively. The four panels represent four different instances where mixed infection was detected. Panels a, c and d contain the two species in relatively large ratios (20-50% mixtures), whereas panel b shows detection of a low abundant (2-5%) mixture. Two specific instances where the observed mixtures contained two of the circulating genotypes (notated as A and D or as A and N) from 2005-06, are shown in panels c and d, respectively.

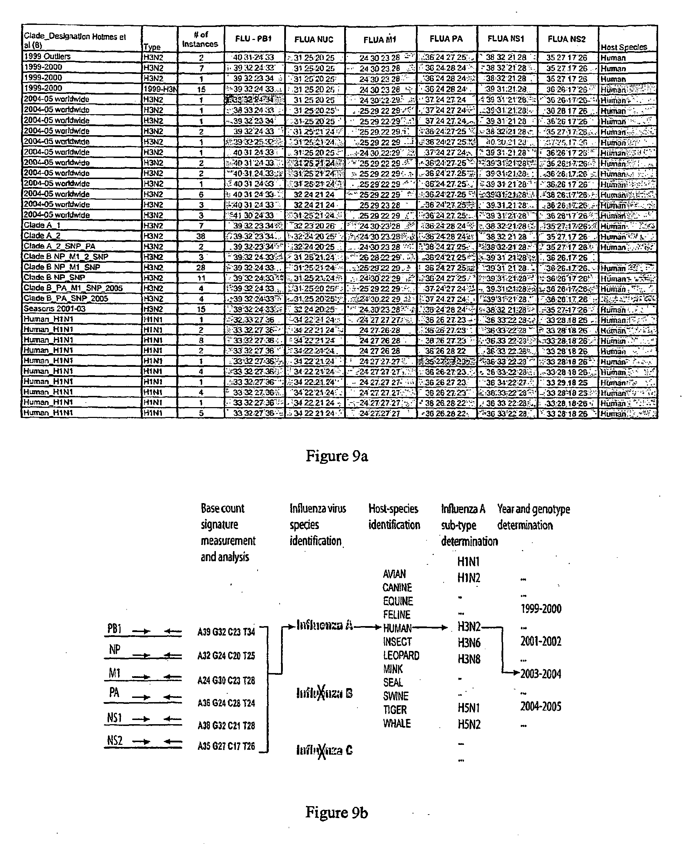

**Figures 9a and 9b****.** PCR-ESI/MS genotyping and tracking of influenza viruses. Figure 9b shows a genotyping schema. A genotype represents a unique combination of base composition signatures at each of the PCR loci. Figure 9a shows distribution of various influenza A genotypes observed in the samples tested. These genotypes were compared to base composition information available in GenBank and the closest matching strain is shown. The genotypes shown here were consistent with the year of collection and the predominant circulating clades during that year.

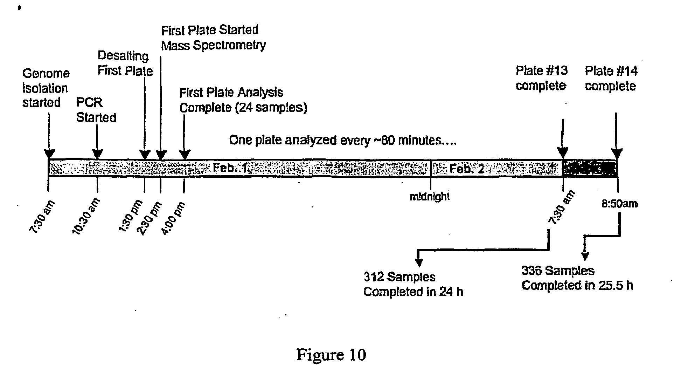

**Figure 10** is a timeline representing high throughput detection and analysis of 336 patient samples comprising an unknown bioagent.

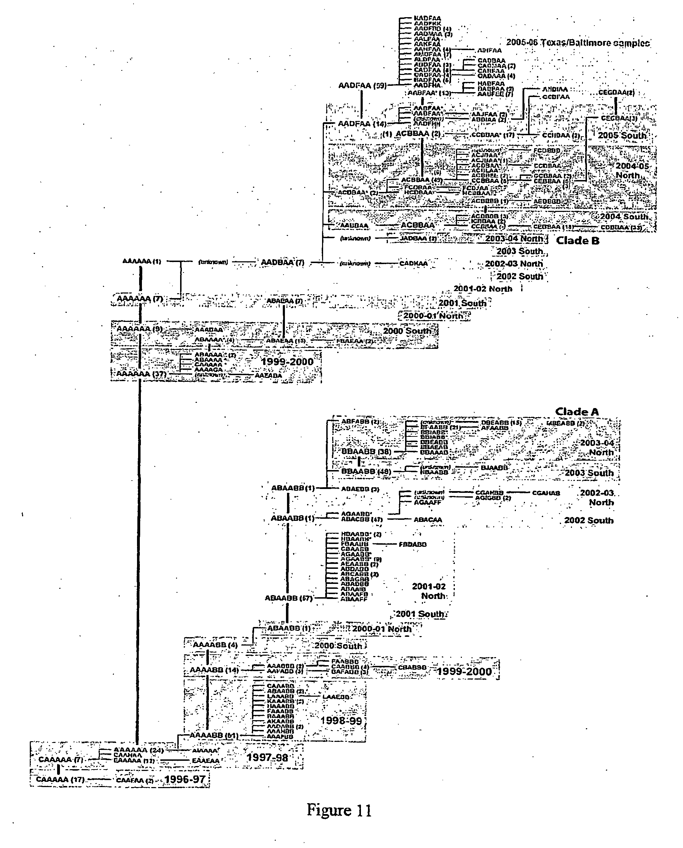

**Figure 11** is an influenza virus clade distribution plot characterizing and tracking the global spread of known influenza virus and the emergence of novel influenza virus. This plot is an analysis of base compositions of influenza virus (H3N2) isolates between years 1996-2006. To capture the geographical sampling location and flu seasons, the isolates were labeled "North" and South" to reflect Northern or Southern hemispheres. Clade A and Clade B designations are based on the analysis by Holmes et al. Vertical Bar: Direct ancestor; Horizontal Bar: Single mutation.

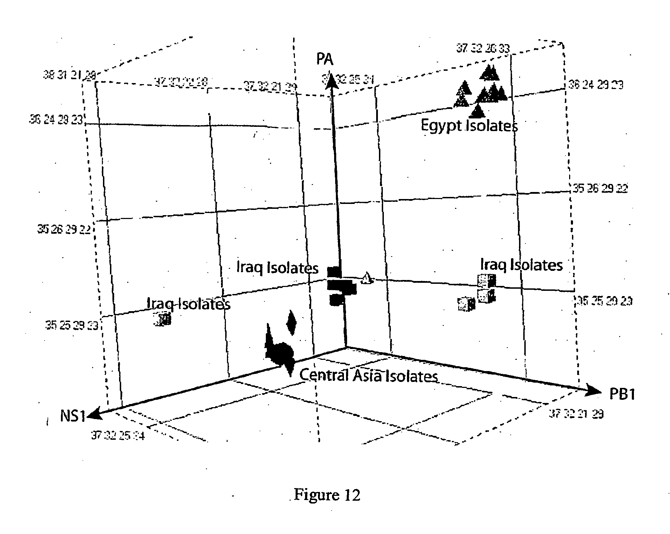

**Figure 12** illustrates a genotype analysis of bioagents based on plotting base composition signature from a plurality of avian influenza A virus H.sub.5N.sub.1 isolates. Bioagents are plotted on the graph as a function of base composition signature. Clusters represent isolates having highly similar genotypes.

### DETAILED DESCRIPTION OF EMBODIMENTS

As is used herein, a "bioagent" means any microorganism or infectious substance, or any naturally occurring, bioengineered or synthesized component of any such microorganism or infectious substance or any nucleic acid derived from any such microorganism or infectious substance. Those of ordinary skill in the art will understand fully what is meant by the term bioagent given the instant disclosure. Still, a non-exhaustive list of bioagents includes: cells, cell lines, human clinical samples, mammalian blood samples, cell cultures, bacterial cells, viruses, viroids, fungi, protists, parasites, rickettsiae or protozoa. Samples may be alive or dead or in a vegetative state (for example, vegetative bacteria or spores). Preferably, the bioagent is a virus or a nucleic acid derived therefrom. More preferably, the bioagent is a member of the *orthomyxovirdae* family, More preferably still the bioagent is an influenzavirus A, B or C.

As used herein, "intelligent primers" or "primers" or "primer pairs" are oligonucleotides that are designed to bind to conserved sequence regions of two or more bioagent nucleic acid to generate bioagent identifying amplicons. The bound primers flank an intervening variable region between the conserved binding sequences. Upon amplification, the primer pairs yield amplicons that provide base composition variability between the two or more bioagents. The variability of the base compositions allows for the identification of one or more individual bioagents from of the two or more bioagents based on the base composition distinctions. The primer pairs are also designed to generate amplicons that are amenable to molecular mass analysis. Primer pair nomenclature, as used herein, includes naming a reference sequence. For example, the forward primer for primer pair number 1259 is named FLUAPB2_NC004518_66_92_F. The reference sequence that this primer is referring to is Gen Bank Accession No: NC_004518 (first entered January 11, 2003). This primer is the forward primer of the pair (as denoted by "_F") and it hybridizes with residues 66-92 of the reference sequence (66_92), the PB2 gene of the referenced influenza A virus. The primer pair are selected and designed; however, to hybridize with two or more bioagents. So, the nomenclature used is merely to provide a reference sequence, and not to indicate that the primers hybridize with and generate a bioagent identifying amplicon only from the reference sequence. Further, the sequences of the primer members of the primer pairs are not necessarily fully complementary to the conserved region oaf the reference bioagent. Rather, the sequences are designed to be "best fit" amongst a plurality of bioagents at these conserved binding sequences. Therefore, the primer members of the primer pairs have substantial complementarity with the conserved regions of the bioagents, including the reference bioagent

As is used herein, the term "substantial complementarity" means that a primer member of a primer pair comprises between about 70%-100%, or between about 80-100%, or between about 90-100%, or between about 95-100% identity, or between about 99-100% sequence identity with the conserved binding sequence of any given bioagent. These ranges of identity are inclusive of all whole or partial numbers embraced within the recited range numbers. For example, and not limitation, 75.667%, 82%, 91.2435% and 97% sequence identity are all numbers that fall within the above recited range of 70% to 100%, therefore forming a part of this description.

As used herein, "broad range survey primers" are intelligent primers designed to identify an unknown bioagent as a member of a particular division (e.g., an order, family, class, clade, or genus). However, in some cases the broad range survey primers are also able to identify unknown bioagents at the species or sub-species level. As used herein, "division-wide primers" are intelligent primers designed to identify a bioagent at the species level and "drill-down" primers are intelligent primers designed to identify a bioagent at the sub-species level. As used herein, the "sub-species" level of identification includes, but is not limited to, strains, subtypes, variants, and isolates. Preferably, and without limitation, the family is *orthomyxovirdae;* the genus is influenzevirus A, influenzavirus B, influenzavirus C, Isavirus or thogotovirus; the species is influenza A virus, influenza B virus or influenza C virus; the sub-species is H.sub.xN.sub.Y subtype and/or is a strain reference notation. Drill-down primers are not always required for identification at the sub-species level because broad range survey intelligent primers may, in some cases provide sufficient identification resolution to accomplishing this identification objective.

As used herein, the term "variable region" is used to describe a region that falls between any one primer pair described herein. The region possesses distinct base compositions between at least two bioagents, such that at least one bioagent can be identified at the family, genus, species or sub-species level. The degree of variability between the at least two bioagents need only be sufficient to allow for identification using mass spectrometry analysis, as described herein. Such differences can be as slight as a single nucleotide difference occurring between two bioagents.

As used herein, the terms "amplicon" or "bioagent identifying amplicon" refer to a nucleic acid generated using the primer pairs described herein. The amplicon is preferably double stranded DNA; however, it may be RNA and/or DNA:RNA. The amplicon comprises the sequences of the conserved regions/primer pairs and the intervening variable region. As discussed herein, primer pairs are designed to generate amplicons from two or more bioagents. As such, the base composition of any given amplicon will include the primer pair, the complement of the primer pair, the conserved regions and the variable region from the bioagent that was amplified to generate the amplicon. One skilled in the art understands that the incorporation of the designed primer pair sequences into any amplicon will replace the native viral sequences at the primer binding site, and complement thereof. After amplification of the target region using the primers the resultant amplicons having the primer sequences generate the molecular mass data. Amplicons having any native viral sequences at the primer binding sites, or complement thereof, are undetectable because of their low abundance. Such is accounted for when identifying one or more bioagents using any particular primer pair. The amplicon further comprises a length that is compatible with mass spectrometry analysis. Bioagent identifying amplicons generate base composition signatures that are preferably unique to the identity of a bioagent.

Calculation of base composition from a mass spectrometer generated molecular mass becomes increasingly more complex as the length of the amplicon increases. For amplicons comprising unmodified nucleic acid, the upper length as a practical length limit is about 200 consecutive nucleobases. Incorporating modified nucleotides into the amplicon can allow for an increase in this upper limit. In one embodiment, the amplicons generated using any single primer pair will provide sufficient base composition information to allow for identification of at least one bioagent at the family, genus, species or subspecies level.

Preferably, amplicons comprise from about 45 to about 200 consecutive nucleobases (i.e., from about 45 to about 200 linked nucleosides). One of ordinary skill in the art will appreciate that this range expressly embodies compounds of 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165,166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, and 200 nucleobases in length. One ordinarily skilled in the art will further appreciate that the above range is not an absolute limit to the length of an amplicon, but instead represents a preferred length range. Amplicons lengths falling outside of this range are also included herein so long as the amplicon is amenable to calculation of a base composition signature as herein described.

As is used herein, the term "unknown bioagent" can mean either: (i) a bioagent whose existence is not known (for example, the SARS coronavirus was unknown prior to April 2003) and/or (ii) a bioagent whose existence is known (such as the well known bacterial species *Staphylococcus aureus* for example) but which is not known to be in a sample to be analyzed. For example, if the method for identification of coronaviruses disclosed in commonly owned U.S. Patent Serial No. 10/829,826 (incorporated herein by reference in its entirety) was to be employed prior to April 2003 to identify the SARS coronavirus in a clinical sample, both meanings of "unknown" bioagent are applicable since the SARS coronavirus was unknown to science prior to April, 2003 and since it was not known what bioagent (in this case a coronavirus) was present in the sample. On the other hand, if the method of U.S. Patent Serial No. 10/829,826 was to be employed subsequent to April 2003 to identify the SARS coronavirus in a clinical sample, only the second meaning (ii) of "unknown" bioagent would apply because the SARS coronavirus became known to science subsequent to April 2003 but because it was not known what bioagent was present in the sample.

As used herein, the term "molecular mass" refers to the mass of a compound as determined using mass spectrometry. Herein, the compound is preferably a nucleic acid, more preferably a double stranded nucleic acid, still more preferably a double stranded DNA nucleic acid and is most preferably an amplicon. When the nucleic acid is double stranded the molecular mass is determined for both strands. Here, the strands are separated either before introduction into the mass spectrometer, or the strands are separated by the mass spectrometer (for example, electro-spray ionization will separate the hybridized strands). The molecular mass of each strand is measured by the mass spectrometer.

As used herein, the term "base composition" refers to the number of each residue comprising an amplicon, without consideration for the linear arrangement of these residues in the strand(s) of the amplicon. The amplicon residues comprise, adenosine (A), guanosine (G), cytidine, (C), (deoxy)thymidine (T), uracil (U), inosine (I), nitroindoles such as 5-nitroindole or 3-nitropyrrole, dP or dK (Hill *et al*.), an acyclic nucleoside analog containing 5-nitroindazole (Van Aerschot et al., Nucleosides and Nucleotides, 1995, 14, 1053-1056), the purine analog 1-(2-deoxy-.beta.-D-ribofuranosyl)-imidazole-4-carboxamide, 2,6-diaminopurine, 5-propynyluracil, 5-propynylcytosine, phenoxazines, including G-clamp, 5-propynyl deoxy-cytidine, deoxy-thymidine nucleotides, 5-propynylcytidine, 5-propynyluridine and mass tag modified versions thereof , including 7-deaza-2'-deoxyadenosine-5-triphosphate, 5-iodo-2'-deoxyuridine-5'-triphosphate, 5-bromo-2'-deoxyuridine-5'-triphosphate, 5-bromo-2'-deoxycytidine-5'-triphosphate, 5-iodo-2'-deoxycytidine-5'-triphosphate, 5-hydroxy-2'-deoxyuridine-5'-triphosphate, 4-thiothymidine-5'-triphosphate, 5-aza-2'-deoxyuridine-5'-triphosphate, 5-fluoro-2'-deoxyuridine-5'-triphosphate, O6-methyl-2'-deoxyguanosine-5'-triphosphate, N2-methyl-2'-deoxyguanosine-5'-triphosphate, 8-oxo-2'-deoxyguanosine-5'-triphosphate or thiothymidine-5'-triphosphate. In some embodiments, the mass-modified nucleobase comprises 15.sup.N or 13.sup.C or both 15.sup.N and 13.sup.C. Preferably, the non-natural nucleosides used herein include 5-propynyluracil, 5-propynylcytosine and inosine. Herein the base composition for an unmodified DNA amplicon is notated as A.sub.wG.sub.xC.sub.yT.sub.z, wherein w, x, y and z are each independently a whole number representing the number of said nucleoside residues in an amplicon. Base compositions for amplicons comprising modified nucleosides are similarly notated to indicate the number of said natural and modified nucleosides in an amplicon. Base compositions are calculated from a molecular mass measurement of an amplicon, as described below. The calculated base composition for any given amplicon is then compared to a database of base compositions. A match between the calculated base composition and a single database entry reveals the identity of the bioagent.

As is used herein, the term "base composition signature" refers to the base composition generated by any one particular amplicon.

As used herein, a "base composition probability cloud" is a representation of the diversity in base composition resulting from a variation in sequence that occurs among different isolates of a given species, family or genus. Base composition calculations for a plurality of amplicons are mapped on a pseudo four-dimensional plot. Related members in a family, genus or species typically cluster within this plot, forming a base composition probability cloud.

As used herein, the term "database" is used to refer to a collection of base composition data. The base composition data in the database is indexed to bioagents and to primer pairs. The base composition data reported in the database comprises the number of each nucleoside in an amplicon that would be generated for each bioagent using each primer. The database can be populated by empirical data. In this aspect of populating the database, a bioagent is selected and a primer pair is used to generate an amplicon. The amplicon's molecular mass is determined using a mass spectrometer and the base composition calculated therefrom. An entry in the database is made to associate the base composition with the bioagent and the primer pair used. The database may also be populated using other databases comprising bioagent information. For example, using the GenBank database it is possible to perform electronic PCR using an electronic representation of a primer pair. This *in silico* method will provide the base composition for any or all selected bioagent(s) stored in the GenBank database. The information is then used to populate the base composition database as described above. A base composition database can be *in silico,* a written table, a reference book, a spreadsheet or any form generally amenable to databases. Preferably, it is *in silico.*

As used herein, the term "nucleobase" is synonymous with other terms in use in the art including "nucleotide," "deoxynucleotide," "nucleotide residue," "deoxynucleotide residue," "nucleotide triphosphate (NTP)," or deoxynucleotide triphosphate (dNTP). As is used herein, a nucleobase includes natural and modified residues, as described herein.

In the context of the present description, "viral nucleic acid" includes, but is not limited to, DNA, RNA, or DNA that has been obtained from viral RNA, such as, for example, by performing a reverse transcription reaction. Viral RNA can either be single-stranded (of positive or negative polarity) or double-stranded.

As used herein, a "wobble base" is a variation in a codon found at the third nucleotide position of a DNA triplet. Variations in conserved regions of sequence are often found at the third nucleotide position due to redundancy in the amino acid code.

As used herein, "housekeeping gene" or "core viral gene" refers to a gene encoding a protein or RNA involved in basic functions required for survival and reproduction of a bioagent. Housekeeping genes include, but are not limited to, genes encoding RNA or proteins involved in translation, replication, recombination and repair, transcription, nucleotide metabolism, amino acid metabolism, lipid metabolism, energy generation, uptake, secretion and the like. Preferably, the core viral genes discussed herein are polymerases (PB1, PB2 and PA), nucleoprotein (NUC or NP), matrix protein (M or M1), non-structural proteins (NS1 and NS2), and glycoproteins (HA and NA).

As used herein, a "bioagent division" is defined as group of bioagents above the species level and includes but is not limited to, orders, families, genus, classes, clades, genera or other such groupings of bioagents above the species level.

As used herein, a "sub-species characteristic" is a genetic characteristic that provides the means to distinguish two members of the same bioagent species. For example, one viral strain could be distinguished from another viral strain of the same species by possessing a genetic change (e.g., for example, a nucleotide deletion, addition or substitution) in one of the viral genes, such as the RNA-dependent RNA polymerase.

As used herein, "triangulation identification" means the employment of more than one primer pair to generate a corresponding amplicon for identification of a bioagent. The more than one primer pair can be used in individual wells or in a multiplex PCR assay. Alternatively, PCR reaction may be carried out in single wells comprising a different primer pair in each well. Following amplification the amplicons are pooled into a single well or container which is then subjected to molecular mass analysis. The combination of pooled amplicons can be chosen such that the expected ranges of molecular masses of individual amplicons are not overlapping and thus will not complicate identification of signals. Triangulation works as a process of elimination, wherein a first primer pair identifies that an unknown bioagent may be one of a group of bioagents. Subsequent primer pairs are used in triangulation identification to further refine the identity of the bioagent amongst the subset of possibilities generated with the earlier primer pair. Triangulation identification is complete when the identity of the bioagent is determined. The triangulation identification process is also used to reduce false negative and false positive signals, and enable reconstruction of the origin of hybrid or otherwise engineered bioagents. For example, identification of the three part toxin genes typical of *B. anthracis* (Bowen et al., J. Appl. Microbiol., 1999, 87, 270-278) in the absence of the expected signatures from the *B. anthracis* genome would suggest a genetic engineering event.

As is used herein, the term "single primer pair identification" means that one or more bioagents can be identified using a single primer pair. A base composition signature for an amplicon may singly identify one or more bioagents.

As used herein, the term "etiology" refers to the causes or origins, of diseases or abnormal physiological conditions.

Provided herein are methods for detection and identification of bioagents in an unbiased manner using bioagent identifying amplicons. Primers are selected to hybridize to conserved sequence regions of nucleic acids derived from a bioagent and which bracket variable sequence regions to yield a bioagent identifying amplicon which can be amplified and which is amenable to molecular mass determination. The molecular mass is converted to a base composition, which indicates the number of each nucleotide in the amplicon. The molecular mass or corresponding base composition signature of the amplicon is then queried against a database of molecular masses or base composition signatures indexed to bioagents and to the primer pair used to generate the amplicon. A match of the measured base composition to a database entry base composition associates the sample bioagent to an indexed bioagent in the database. Thus the identity of the unknown bioagent is determined. Prior knowledge of the unknown bioagent is not necessary. In some instances, the measured base composition associates with more than one database entry base composition. Thus, a second/subsequent primer pair is used to generate an amplicon, and its measured base composition is similarly compared to the database to determine its identity in triangulation identification. Furthermore, the method can be applied to rapid parallel multiplex analyses, the results of which can be employed in a triangulation identification strategy. The present method provides rapid throughput and does not require nucleic acid sequencing of the amplified target sequence for bioagent detection and identification.

Despite enormous biological diversity, all forms of life on earth share sets of essential, common features in their genomes. Since genetic data provide the underlying basis for identification of bioagents by the current methods, it is necessary to select segments of nucleic acids which ideally provide enough variability to distinguish each individual bioagent and whose molecular mass is amenable to molecular mass determination.

Unlike bacterial genomes, which exhibit conservation of numerous genes (i.e. housekeeping genes) across all organisms, viruses do not share a single gene that is essential and conserved among all virus families. Therefore, viral identification is achieved within smaller groups of related viruses, such as members of a particular virus family or genus. For example, RNA-dependent RNA polymerase is present in all single-stranded RNA viruses and can be used for broad priming as well as resolution within the virus family.

In some embodiments, at least one viral nucleic acid segment is amplified in the process of identifying the bioagent. Thus, the nucleic acid segments that can be amplified by the primers disclosed herein and that provide enough variability to distinguish each individual bioagent and whose molecular masses are amenable to molecular mass determination are herein described as bioagent identifying amplicons.

It is the combination of the portions of the bioagent nucleic acid segment to which the primers hybridize (hybridization sites) and the variable region between the primer hybridization sites that comprises the bioagent identifying amplicon.

In some embodiments, bioagent identifying amplicons amenable to molecular mass determination which are produced by the primers described herein are either of a length, size or mass compatible with the particular mode of molecular mass determination or compatible with a means of providing a predictable fragmentation pattern in order to obtain predictable fragments of a length compatible with the particular mode of molecular mass determination. Such means of providing a predictable fragmentation pattern of an amplicon include, but are not limited to, cleavage with restriction enzymes or cleavage primers, for example. Thus, in some embodiments, bioagent identifying amplicons are larger than 200 nucleobases and are amenable to molecular mass determination following restriction digestion. Methods of using restriction enzymes and cleavage primers are well known to those with ordinary skill in the art.

In some embodiments, amplicons corresponding to bioagent identifying amplicons are obtained using the polymerase chain reaction (PCR) which is a routine method to those with ordinary skill in the molecular biology arts. Other amplification methods may be used such as ligase chain reaction (LCR), low-stringency single primer PCR, and multiple strand displacement amplification (MDA). These methods are also known to those with ordinary skill. (Michael, SF., Biotechniques (1994), 16:411-412 and Dean et al., Proc. Natl. Acad. Sci. U.S.A. (2002), 99,5261-5266)

A representative process flow diagram used for primer selection and validation process is outlined in Figure 1. For each group of organisms, candidate target sequences are identified (200) from which nucleotide alignments are created (210) and analyzed (220). Primers are then designed by selecting appropriate priming regions (230) to facilitate the selection of candidate primer pairs (240). The primer pair sequence is a "best fit" amongst the aligned sequences, meaning that the primer pair sequence may or may not be fully complementary to the hybridization region on any one of the bioagents in the alignment. Thus, bets fit primer pair sequences are those with sufficient complementarity with two or more bioagents to hybridize with the two or more bioagents and generate an amplicon. The primer pairs are then subjected to *in silico* analysis by electronic PCR (ePCR) (300) wherein bioagent identifying amplicons are obtained from sequence databases such as Genbank or other sequence collections (310) and checked for specificity *in silico* (320). Bioagent identifying amplicons obtained from ePCR of GenBank sequences (310) can also be analyzed by a probability model which predicts the capability of a given amplicon to identify unknown bioagents. Preferably, the base compositions of amplicons with favorable probability scores are then stored in a base composition database (325). Alternatively, base compositions of the bioagent identifying amplicons obtained from the primers and GenBank sequences can be directly entered into the base composition database (330). Candidate primer pairs (240) are validated by *in* vitro amplification by a method such as PCR analysis (400) of nucleic acid from a collection of organisms (410). Amplicons thus obtained are analyzed to confirm the sensitivity, specificity and reproducibility of the primers used to obtain the amplicons (420).

Synthesis of primers is well known and routine in the art. The primers may be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed.

The primers are employed as compositions for use in methods for identification of viral bioagents as follows: a primer pair composition is contacted with nucleic acid (such as, for example, DNA from a DNA virus, or DNA reverse transcribed from the RNA of an RNA virus) of an unknown viral bioagent. The nucleic acid is then amplified by a nucleic acid amplification technique, such as PCR for example, to obtain an amplicon that represents a bioagent identifying amplicon. The molecular mass of each strand of the double-stranded amplicon is determined by a molecular mass measurement technique such as mass spectrometry for example. Preferably the two strands of the double-stranded amplicon are separated during the ionization process; however, they may be separated prior to mass spectrometry measurement. In some embodiments, the mass spectrometer is electrospray Fourier transform ion cyclotron resonance mass spectrometry (ESI-FTICR-MS) or electrospray time of flight mass spectrometry (ESI-TOF-MS). A list of possible base compositions can be generated for the molecular mass value obtained for each strand and the choice of the correct base composition from the list is facilitated by matching the base composition of one strand with a complementary base composition of the other strand. The measured molecular mass or base composition calculated therefrom is then compared with a database of molecular masses or base compositions indexed to primer pairs and to known viral bioagents. A match between the measured molecular mass or base composition of the amplicon and the database molecular mass or base composition for that indexed primer pair will associate the measured molecular mass or base composition with an indexed viral bioagent, thus indicating the identity of the unknown bioagent. In some embodiments, the primer pair used is one of the primer pairs of Table 2. In some embodiments, the method is repeated using a different primer pair to resolve possible ambiguities in the identification process or to improve the confidence level for the identification assignment (triangulation identification).

In some embodiments, a bioagent identifying amplicon may be produced using only a single primer (either the forward or reverse primer of any given primer pair), provided an appropriate amplification method is chosen, such as, for example, low stringency single primer PCR (LSSP-PCR). Adaptation of this amplification method in order to produce bioagent identifying amplicons can be accomplished by one with ordinary skill in the art without undue experimentation. (Pena, SDJ et al., Proc. Natl. Acad. Sci. U.S.A (1994) 91, 1946-1949).

In some embodiments, the oligonucleotide primers are broad range survey primers which hybridize to conserved regions of nucleic acid encoding the PB1 gene or the NUC gene, a gene that is common to all known influenza viruses, though the sequences vary. The broad range primer may identify the unknown bioagent, depending on which bioagent is in the sample. In other cases, the molecular mass or base composition of an amplicon does not provide enough resolution to unambiguously identify the unknown bioagent as any one viral bioagent at or below the species level. These cases benefit from further analysis of one or more an amplicons generated from at least one additional broad range survey primer pair or from at least one additional division-wide primer pair or from at least one additional drill-down primer pair. Identification of sub-species characteristics is often critical for determining proper clinical treatment of viral infections, or in rapidly responding to an outbreak of a new viral strain to prevent massive epidemic or pandemic.

In some embodiments, the primers used for amplification hybridize to and amplify genomic DNA, DNA of bacterial plasmids, DNA of DNA viruses or DNA reverse transcribed from RNA of an RNA virus. Among other things, the identification of non-viral nucleic acids or combinations of viral and non-viral nucleic acids are useful for detecting bioengineered bioagents.

In some embodiments, the primers used for amplification hybridize directly to viral RNA and act as reverse transcription primers for obtaining DNA from direct amplification of viral RNA. Methods of amplifying RNA to produce cDNA using reverse transcriptase are well known to those with ordinary skill in the art and can be routinely established without undue experimentation.

One with ordinary skill in the art of design of amplification primers will recognize that a given primer need not hybridize with 100% complementarity in order to effectively prime the synthesis of a complementary nucleic acid strand in an amplification reaction. Primer pair sequences may be a "best fit" amongst the aligned bioagent sequences, thus not be fully complementary to the hybridization region on any one of the bioagents in the alignment. Moreover, a primer may hybridize over one or more segments such that intervening or adjacent segments are not involved in the hybridization event. (e.g., for example, a loop structure or a hairpin structure). The primers may comprise at least 70%, at least 75%, at least 80%, at least 85%, at least 90%, at least 95% or at least 99% sequence identity with any of the primers listed in Table 2. Thus, in some embodiments, an extent of variation of 70% to 100%, or any range falling within, of the sequence identity is possible relative to the specific primer sequences disclosed herein. Determination of sequence identity is described in the following example: a primer 20 nucleobases in length which is identical to another 20 nucleobase primer having two non-identical residues has 18 of 20 identical residues (18/20 = 0.9 or 90% sequence identity). In another example, a primer 15 nucleobases in length having all residues identical to a 15 nucleobase segment of primer 20 nucleobases in length would have 15/20 = 0.75 or 75% sequence identity with the 20 nucleobase primer. Percent identity need not be a whole number, for example when a 28 consecutive nucleobase primer is completely identical to a 31 consecutive nucleobase primer (28/31 = 0.9032 or 90.3% identical).

Percent homology, sequence identity or complementarity, can be determined by, for example, the Gap program (Wisconsin Sequence Analysis Package, Version 8 for Unix, Genetics Computer Group, University Research Park, Madison WI), using default settings, which uses the algorithm of Smith and Waterman (Adv. Appl. Math., 1981, 2, 482-489). In some embodiments, complementarity of primers with respect to the conserved priming regions of viral nucleic acid, is between about 70% and about 80%. In other embodiments, homology, sequence identity or complementarity, is between about 80% and about 90%. In yet other embodiments, homology, sequence identity or complementarity, is at least 90%, at least 92%, at least 94%, at least 95%, at least 96%, at least 97%, at least 98%, at least 99% or is 100%.

In some embodiments, the primers described herein comprise at least 70%, at least 75%, at least 80%, at least 85%, at least 90%, at least 92%, at least 94%, at least 95%, at least 96%, at least 98%, or at least 99%, or 100% (or any range falling within) sequence identity with the primer sequences specifically disclosed herein.

One with ordinary skill is able to calculate percent sequence identity or percent sequence homology and is able to determine, without undue experimentation, the effects of variation of primer sequence identity on the function of the primer in its role in priming synthesis of a complementary strand of nucleic acid for production of an amplicon of a corresponding bioagent identifying amplicon.

In some embodiments, the oligonucleotide primers are 13 to 35 nucleobases in length (13 to 35 linked nucleotide residues). These embodiments comprise oligonucleotide primers 13, 14, 15, 16, 17, 18, 19, 20, 21, 22, 23, 24, 25, 26, 27, 28, 29, 30, 31, 32, 33, 34 or 35 nucleobases in length, or any range therewithin.

In some embodiments, any given primer comprises a modification comprising the addition of a non-templated T residue to the 5' end of the primer (i.e., the added T residue does not necessarily hybridize to the nucleic acid being amplified). The addition of a non-templated T residue has an effect of minimizing the addition of non-templated A residues as a result of the non-specific enzyme activity of *Taq* polymerase (Magnuson et al., Biotechniques, 1996, 21, 700-709), an occurrence which may lead to ambiguous results arising from molecular mass analysis.

Primers may contain one or more universal bases. Because any variation (due to codon wobble in the third position) in the conserved regions among species is likely to occur in the third position of a DNA (or RNA) triplet, oligonucleotide primers can be designed such that the nucleotide corresponding to this position is a base which can bind to more than one nucleotide, referred to herein as a "universal nucleobase." For example, under this "wobble" pairing, inosine (I) binds to U, C or A; guanine (G) binds to U or C, and uridine (U) binds to U or C. Other examples of universal nucleobases include nitroindoles such as 5-nitroindole or 3-nitropyrrole (Loakes et al., Nucleosides and Nucleotides, 1995, 14, 1001-1003), the degenerate nucleotides dP or dK (Hill *et al.),* an acyclic nucleoside analog containing 5-nitroindazole (Van Aerschot et al., Nucleosides and Nucleotides, 1995, 14, 1053-1056) or the purine analog 1-(2-deoxy-beta.-D-ribofuranosyl)-imidazole-4-carboxamide (Sala et al., Nucl. Acids Res., 1996, 24, 3302-3306).

In some embodiments, to compensate for the somewhat weaker binding by the wobble base, the oligonucleotide primers are designed such that the first and second positions of each triplet are occupied by nucleotide analogs which bind with greater affinity than the unmodified nucleotide. Examples of these analogs include, but are not limited to, 2,6-diaminopurine which binds to thymine, 5-propynyluracil which binds to adenine and 5-propynylcytosine and phenoxazines, including G-clamp, which binds to G. Propynylated pyrimidines are described in U.S. Patent Nos. 5,645,985, 5,830,653 and 5,484,908. Propynylated primers are described in U.S Pre-Grant Publication No. 2003-0170682. Phenoxazines are described in U.S. Patent Nos. 5,502,177, 5,763,588, and 6,005,096. G-clamps are described in U.S. Patent Nos. 6,007,992 and 6,028,183.

In some embodiments, to enable broad priming of rapidly evolving RNA viruses, primer hybridization is enhanced using primers and probes containing 5-propynyl deoxy-cytidine and deoxy-thymidine nucleotides. These modified primers offer increased affinity and base pairing selectivity.

In some embodiments, non-template primer tags are used to increase the melting temperature (T.sub.m) of a primer-template duplex in order to improve amplification efficiency. A non-template tag is at least three consecutive A or T nucleotide residues on a primer which are not complementary to the template. In any given non-template tag, A can be replaced by C or G and T can also be replaced by C or G. Although Watson-Crick hybridization is not expected to occur for a non-template tag relative to the template, the extra hydrogen bond in a G-C pair relative to an A-T pair confers increased stability of the primer-template duplex and improves amplification efficiency for subsequent cycles of amplification when the primers hybridize to strands synthesized in previous cycles.

In other embodiments, propynylated tags may be used in a manner similar to that of the non-template tag, wherein two or more 5-propynylcytidine or 5-propynyluridine residues replace template matching residues on a primer. In other embodiments, a primer contains a modified internucleoside linkage such as a phosphorothioate linkage, for example.

In some embodiments, the primers contain mass-modifying tags. Reducing the total number of possible base compositions of a nucleic acid of specific molecular weight provides a means of avoiding a persistent source of ambiguity in determination of base composition of amplicons. Addition of mass-modifying tags to certain nucleobases of a given primer will result in simplification of *de novo* determination of base composition of a given bioagent identifying amplicon from its molecular mass.

In some embodiments, the mass modified nucleobase comprises one or more of the following: for example, 7-deaza-2'-deoxyadenosine-5-triphosphate, 5-iodo-2'-deoxyuridine-5'-triphosphate, 5-bromo-2'-deoxyuridine-5'-triphosphate, 5-bromo-2'-deoxycytidine-5'-triphosphate, 5-iodo-2'-deoxycytidine-5'-triphosphate, 5-hydroxy-2'-deoxyuridine-5'-triphosphate, 4-thiothymidine-5'-triphosphate, 5-aza-2'-deoxyuridine-5'-triphosphate, 5-fluoro-2'-deoxyuridine-5'-triphosphate, O6-methyl-2'-deoxyguanosine-5'-triphosphate, N2-methyl-2'-deoxyguanosine-5'-triphosphate, 8-oxo-2'-deoxyguanosine-5'-triphosphate or thiothymidine-5'-triphosphate. In some embodiments, the mass-modified nucleobase comprises .sup.15N or .sup.13C or both .sup.15N and .sup.13C.

In some embodiments, the molecular mass of a given bioagent identifying amplicon is determined by mass spectrometry. Mass spectrometry has several advantages, not the least of which is high bandwidth characterized by the ability to separate (and isolate) many molecular peaks across a broad range of mass to charge ratio (m/z). Thus mass spectrometry is intrinsically a parallel detection scheme without the need for radioactive or fluorescent labels, since every amplicon is identified by its molecular mass. The current state of the art in mass spectrometry is such that less than femtomole quantities of material can be readily analyzed to afford information about the molecular contents of the sample. An accurate assessment of the molecular mass of the material can be quickly obtained, irrespective of whether the molecular weight of the sample is several hundred, or in excess of one hundred thousand atomic mass units (amu) or Daltons.

In some embodiments, intact molecular ions are generated from amplicons using one of a variety of ionization techniques to convert the sample to gas phase. These ionization methods include, but are not limited to, electrospray ionization (ES), matrix-assisted laser desorption ionization (MALDI) and fast atom bombardment (FAB). Upon ionization, several peaks are observed from one sample due to the formation of ions with different charges. Averaging the multiple readings of molecular mass obtained from a single mass spectrum affords an estimate of molecular mass of the bioagent identifying amplicon. Electrospray ionization mass spectrometry (ESI-MS) is particularly useful for very high molecular weight polymers such as proteins and nucleic acids having molecular weights greater than 10 kDa, since it yields a distribution of multiply charged molecules of the sample without causing a significant amount of fragmentation.

The mass detectors used include, but are not limited to, Fourier transform ion cyclotron resonance mass spectrometry (FT-ICR-MS), time of flight (TOF), ion trap, quadrupole, magnetic sector, Q-TOF, and triple quadrupole.

In some embodiments, assignment of previously unobserved base compositions (also known as "true unknown base compositions") to a given phylogeny can be accomplished via the use of pattern classifier model algorithms. Base compositions, like sequences, vary slightly from strain to strain within species, for example. In some embodiments, the pattern classifier model is the mutational probability model. On other embodiments, the pattern classifier is the polytope model.

In one embodiment, it is possible to manage this diversity by building "base composition probability clouds" around the composition constraints for each species. This permits identification of organisms in a fashion similar to sequence analysis. A "pseudo four-dimensional plot" can be used to visualize the concept of base composition probability clouds. Optimal primer design requires optimal choice of bioagent identifying amplicons and maximizes the separation between the base composition signatures of individual bioagents. Areas where clouds overlap indicate regions that may result in a misclassification, a problem which is overcome by a triangulation identification process using bioagent identifying amplicons not affected by overlap of base composition probability clouds.

In some embodiments, base composition probability clouds provide the means for screening potential primer pairs in order to avoid potential misclassifications of base compositions. In other embodiments, base composition probability clouds provide the means for predicting the identity of an unknown bioagent whose assigned base composition was not previously observed and/or indexed in a bioagent identifying amplicon base composition database due to evolutionary transitions in its nucleic acid sequence. Thus, in contrast to probebased techniques, mass spectrometry determination of base composition does not require prior knowledge of the composition or sequence in order to make the measurement.

Provided herein are bioagent classifying information at a level sufficient to identify a given bioagent. Furthermore, the process of determining a previously unknown base composition for a given bioagent (for example, in a case where sequence information is unavailable) has downstream utility by providing additional bioagent indexing information with which to populate base composition databases. The process of future bioagent identification is thus greatly improved as more base composition signature indexes become available in base composition databases.

In some embodiments, the identity and quantity of an unknown bioagent can be determined using the process illustrated in Figure 4. Primers (500) and a known quantity of a calibration polynucleotide (505) is added to a sample containing nucleic acid of an unknown bioagent. The total nucleic acid in the sample is then subjected to an amplification reaction (510) to obtain amplicons. The molecular masses of amplicons are determined (515) from which are obtained molecular mass and abundance data. The molecular mass of the bioagent identifying amplicon (520) provides for its identification (525) and the molecular mass of the calibration amplicon obtained from the calibration polynucleotide (530) provides for its quantification (535). The abundance data of the bioagent identifying amplicon is recorded (540) and the abundance data for the calibration data is recorded (545), both of which are used in a calculation (550) which determines the quantity of unknown bioagent in the sample.

A sample comprising an unknown bioagent is contacted with a primer pair which amplifies the nucleic acid from the bioagent, and a known quantity of a polynucleotide that comprises a calibration sequence. The rate of amplification is reasonably assumed to be similar for the nucleic acid of the bioagent and for the calibration sequence. The amplification reaction then produces two amplicons: a bioagent identifying amplicon and a calibration amplicon. The bioagent identifying amplicon and the calibration amplicon should be distinguishable by molecular mass while being amplified at essentially the same rate. Effecting differential molecular masses can be accomplished by choosing as a calibration sequence, a representative bioagent identifying amplicon (from a specific species of bioagent) and performing, for example, a 2-8 nucleobase deletion or insertion within the variable region between the two priming sites. The amplified sample containing the bioagent identifying amplicon and the calibration amplicon is then subjected to molecular mass analysis by mass spectrometry, for example. The resulting molecular mass analysis of the nucleic acid of the bioagent and of the calibration sequence provides molecular mass data and abundance data for the nucleic acid of the bioagent and of the calibration sequence. The molecular mass data obtained for the nucleic acid of the bioagent enables identification of the unknown bioagent by base composition analysis. The abundance data enables calculation of the quantity of the bioagent, based on the knowledge of the quantity of calibration polynucleotide contacted with the sample.

In some embodiments, construction of a standard curve where the amount of calibration polynucleotide spiked into the sample is varied provides additional resolution and improved confidence for the determination of the quantity of bioagent in the sample. The use of standard curves for analytical determination of molecular quantities is well known to one with ordinary skill and can be performed without undue experimentation. Alternatively, the calibration polynucleotide can be amplified in into own reaction well or wells under the same conditions as the bioagent. A standard curve can be prepared therefrom, and a relative abundance of the bioagent determined by methods such as linear regression. In some embodiments, multiplex amplification is performed where multiple bioagent identifying amplicons are amplified with multiple primer pairs which also amplify the corresponding standard calibration sequences. In this or other embodiments, the standard calibration sequences are optionally included within a single construct (preferably a vector) which functions as the calibration polynucleotide. Competitive PCR, quantitative PCR, quantitative competitive PCR, multiplex and calibration polynucleotides are all methods and materials well known to those ordinarily skilled in the art and can be performed without undue experimentation.

In some embodiments, the calibrant polynucleotide is used as an internal positive control to confirm that amplification conditions and subsequent analysis steps are successful in producing a measurable amplicon. Even in the absence of copies of the genome of a bioagent, the calibration polynucleotide should give rise to a calibration amplicon. Failure to produce a measurable calibration amplicon indicates a failure of amplification or subsequent analysis step such as amplicon purification or molecular mass determination. Reaching a conclusion that such failures have occurred is in itself, a useful event. In some embodiments, the calibration sequence is comprised of DNA. In some embodiments, the calibration sequence is comprised of RNA.

In the preferred embodiment, the calibration sequence is inserted into a vector which then itself functions as the calibration polynucleotide. In some embodiments, more than one calibration sequence is inserted into the vector that functions as the calibration polynucleotide. Such a calibration polynucleotide is herein termed a "combination calibration polynucleotide." The process of inserting polynucleotides into vectors is routine to those skilled in the art and can be accomplished without undue experimentation. Thus, it should be recognized that the calibration method should not be limited to the embodiments described herein. The calibration method can be applied for determination of the quantity of any bioagent identifying amplicon when an appropriate standard calibrant polynucleotide sequence is designed and used. The process of choosing an appropriate vector for insertion of a calibrant is also a routine operation that can be accomplished by one with ordinary skill without undue experimentation.

It is preferable for some primer pairs to produce bioagent identifying amplicons within more conserved regions of influenza viruses while others produce bioagent identifying amplicons within regions that are likely to evolve more quickly. Primer pairs that characterize amplicons in a conserved region with low probability that the region will evolve past the point of primer recognition are useful as a broad range survey-type primer. Primer pairs that characterize an amplicon corresponding to an evolving genomic region are useful for distinguishing emerging strain variants.

The primer pairs described herein establish a platform for identifying diseases caused by emerging viruses. Base composition analysis eliminates the need for prior knowledge of bioagent sequence to generate hybridization probes. Thus, in another embodiment, there is provided a method for determining the etiology of a virus infection when the process of identification of viruses is carried out in a clinical setting and, even when the virus is a new species never observed before. This is possible because the methods are not confounded by naturally occurring evolutionary variations (a major concern when using probe based or sequencing dependent methods for characterizing viruses that evolve rapidly). Measurement of molecular mass and determination of base composition is accomplished in an unbiased manner without sequence prejudice and without the need for specificity as is required with probes.

Another embodiment provides a means of tracking the spread of any species or strain of virus when a plurality of samples obtained from different locations are analyzed by the methods described above in an epidemiological setting. For example, a plurality of samples from a plurality of different locations is analyzed with primers which produce bioagent identifying amplicons, a subset of which contains a specific virus. The corresponding locations of the members of the virus-containing subset indicate the spread of the specific virus to the corresponding locations.

Also provided are kits for carrying out the methods described herein. In some embodiments, the kit may comprise a sufficient quantity of one or more primer pairs to perform an amplification reaction on a target polynucleotide from a bioagent to form a bioagent identifying amplicon. In some embodiments, the kit may comprise from one to fifty primer pairs, from one to twenty primer pairs, from one to ten primer pairs, from one to eight primer pairs or from two to five primer pairs. In some embodiments, the kit may comprise one or more primer pairs recited in Table 2.

In some embodiments, the kit may comprise one or more broad range survey primer(s), division wide primer(s), or drill-down primer(s), or any combination thereof. A kit may be designed so as to comprise select primer pairs for identification of a particular bioagent. For example, a broad range survey primer kit may be used initially to identify an unknown bioagent as a member of the family orthomyxoviridae. Another example of a division-wide kit may be used to distinguish human influenza virus type A from influenza virus type B, or from type C for example. A drill-down kit may be used, for example, to distinguish different serotypes of influenza viruses or genetically engineered influenza viruses. In some embodiments, any of these kits may be combined to comprise a combination of broad range survey primers and division-wide primers so as to be able to identify the influenza virus. In some embodiments, the kit may contain standardized calibration polynucleotides for use as internal amplification calibrants.

In some embodiments, the kit may also comprise a sufficient quantity of reverse transcriptase (if an RNA virus is to be identified for example), a DNA polymerase, suitable nucleoside triphosphates (including any of those described above), a DNA ligase, and/or reaction buffer, or any combination thereof, for the amplification processes described above. A kit may further include instructions pertinent for the particular embodiment of the kit, such instructions describing the primer pairs and amplification conditions for operation of the method. A kit may also comprise amplification reaction containers such as microcentrifuge tubes and the like. A kit may also comprise reagents or other materials for isolating bioagent nucleic acid or bioagent identifying amplicons from amplification, including, for example, detergents, solvents, or ion exchange resins which may be linked to magnetic beads. A kit may also comprise a table of measured or calculated molecular masses and/or base compositions of bioagents using the primer pairs of the kit.

The following examples serve only as illustration, and not limitation.

### EXAMPLES

### Example 1: Selection of Design and Validation of Primers that Define Bioagent Identifying Amplicons for Influenza Viruses

For design of primers that define influenza virus identifying amplicons, a series of influenza virus genome segment sequences were obtained, aligned and scanned for regions where pairs of PCR primers would amplify products of about 45 to about 150 nucleotides in length and distinguish species (influenza viruses A, B and C) and/or individual strains from each other by their molecular masses or base compositions. A typical process shown in Figure 1 is employed for this type of analysis.

A database of expected base compositions for each primer region was generated using an *in silico* PCR search algorithm, such as (ePCR). An existing RNA structure search algorithm (Macke et al., Nucl. Acids Res., 2001, 29, 4724-4735) has been modified to include PCR parameters such as hybridization conditions, mismatches, and thermodynamic calculations (SantaLucia, Proc. Natl. Acad. Sci. U.S.A., 1998, 95, 1460-1465). This also provides information on primer specificity of the selected primer pairs.

In addition to the broad range influenza primers, several other primers specific to the influenza A and influenza B species have also been developed. Table 1 shows the influenza segments that were used for primer design and the specificity of the target viral species. It is worth noting that a six nucleotide deletion in the influenza type B sequence of the PB1 gene helps to differentiate the species from influenza viruses type A and C when influenza virus identifying amplicons are obtained using primer pair no: 1299 (SEQ ID NOs: 81:82), which can also simultaneously identify known influenza strains (human, avian, swine, etc.). Figure 2 is a three dimensional diagram indicating resolution of influenza virus identifying amplicons of the three principal species of influenza viruses as primed by primer pair number 1299.

**Table 1: Numbers of Primer Pairs Targeting M1, NUC, PA, PB2 and PB1 Genes for Amplification of Influenza Virus Species**

| **Segment** | **All Influenza virus Species (A, B and C)** | **Influenza virus Species A** | **Influenza virus Species B** | **Influenza virus Species** | **Total** |
|---|---|---|---|---|---|
| **M1** | - | 3 | 3 | 3 | 9 |
| **NUC** | - | 7 | 3 | 2 | 12 |
| **PA** | - | 4 | 4 | 2 | 10 |
| **PB2** | - | 2 | 2 | 2 | 6 |
| **PB1** | 4 | - | - | - | 4 |
| **Total** | 4 | 16 | 12 | 9 | 41 |

A total of 78 primer pairs were designed, of which five were targeted broadly to all known influenza species (primer names containing "FLU_ALL"). The remaining were species type-specific as shown in Table 2, which is a collection of primers (sorted by forward primer name) designed to identify influenza viruses using the methods described herein. "I" represents inosine. The primer pair number is an in-house database index number. Primer sites were identified on influenza virus genes including PB1, PB2, NUC, M1 and PA. The forward or reverse primer name shown in Table 2 indicates the gene region of the viral genome to which the primer hybridizes relative to a reference sequence. The forward primer name FLUAPB2_NC004518_66_92_F indicates that the forward primer ("_F") hybridizes to residues 66-92 ("66_92")of the PB2 gene ("PB2") of a reference virus. In this example the reference virus is influenza A virus ("FLUA") referenced in GenBank as accession number NC_004518 ("_NUC004518_"). The reference virus nomenclature in the primer name is selected to provide a reference, and does not necessarily mean that the primer pair has been designed with 100% complementarity to that target site on the reference virus. A description of the primer design is provided herein. The term "MODS" refers to a primer pair having at least one modification relative to an earlier designed primer pair. For example, primer pair number 2708 (forward primer name FLUPB1_**1297MODS**_J02151_1212_1235_F) has an inosine substitutions relative to the forward primer of primer pair number 1297.

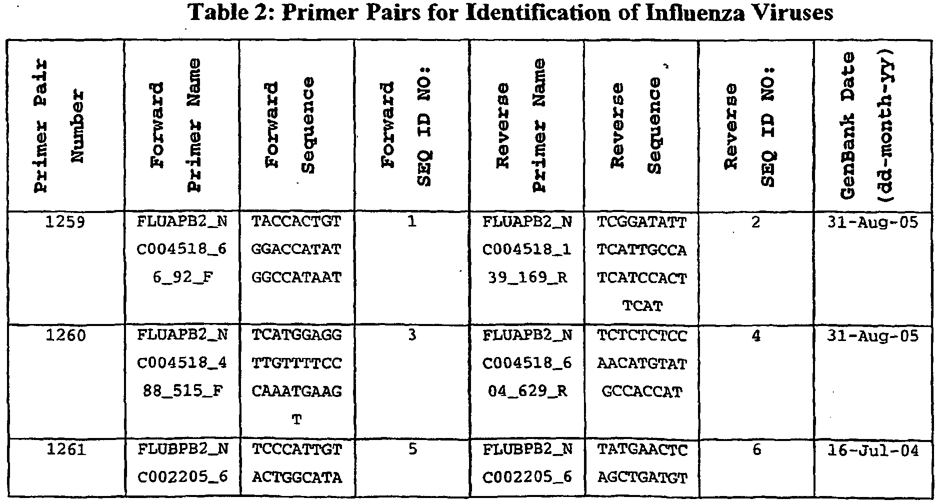

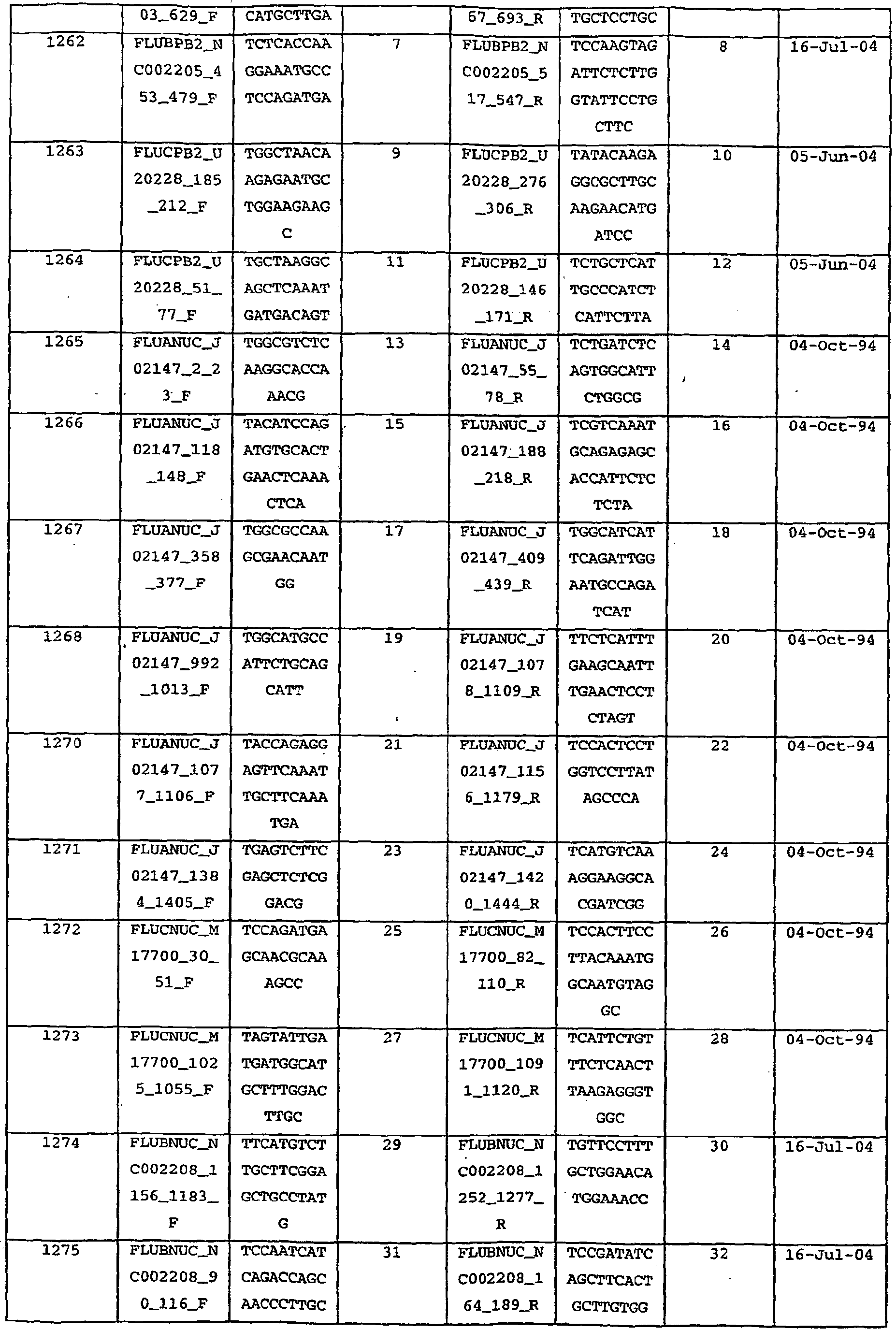

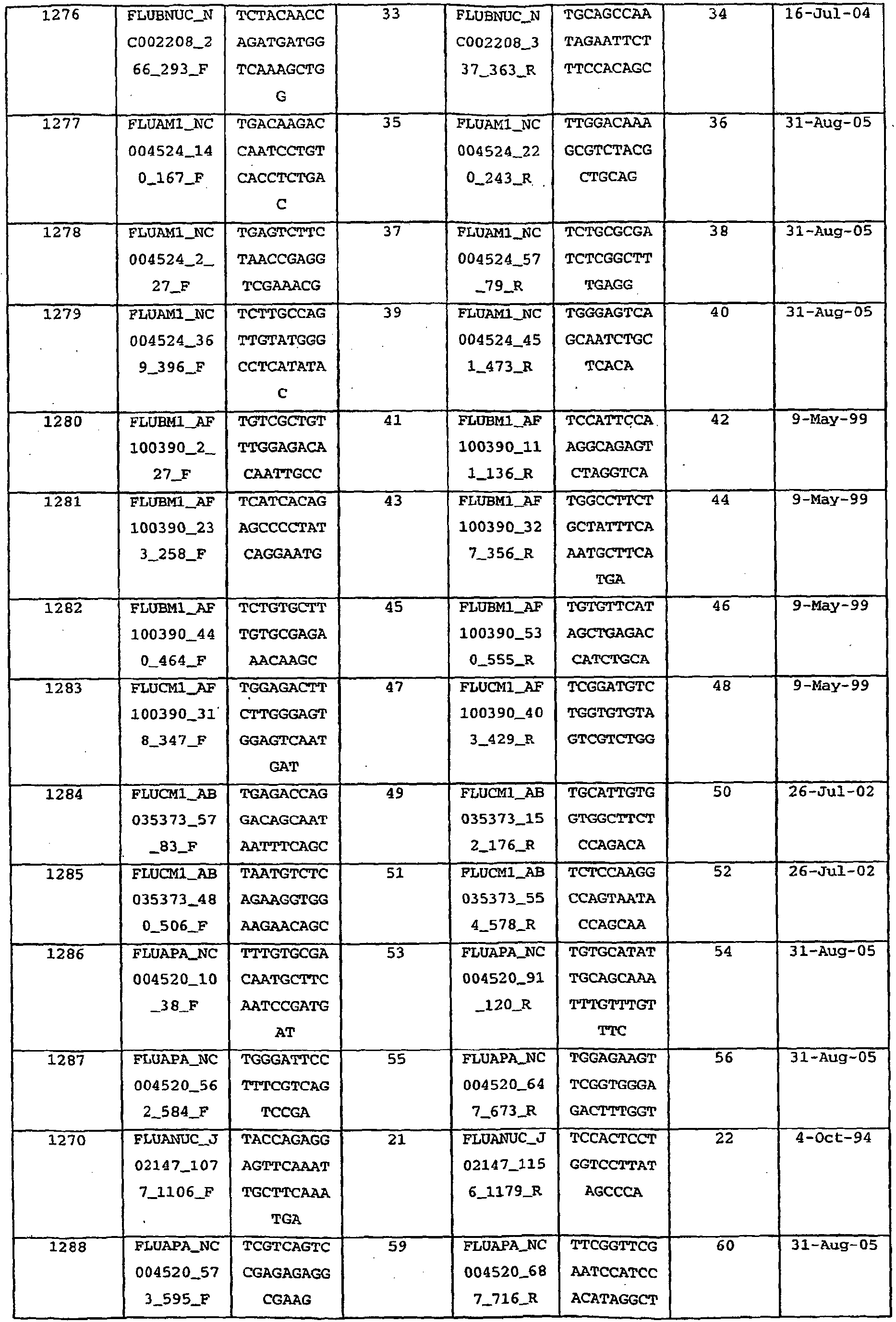

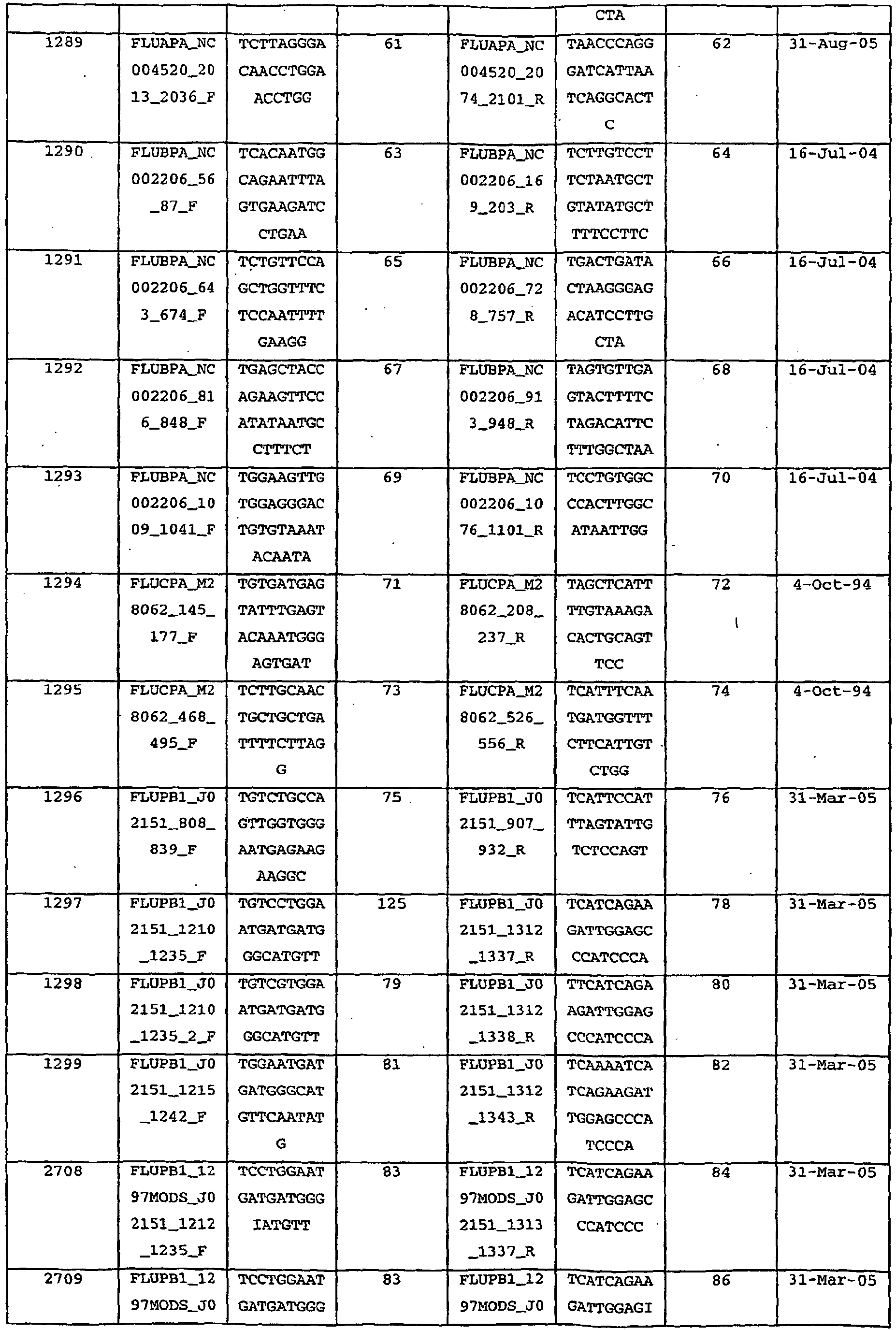

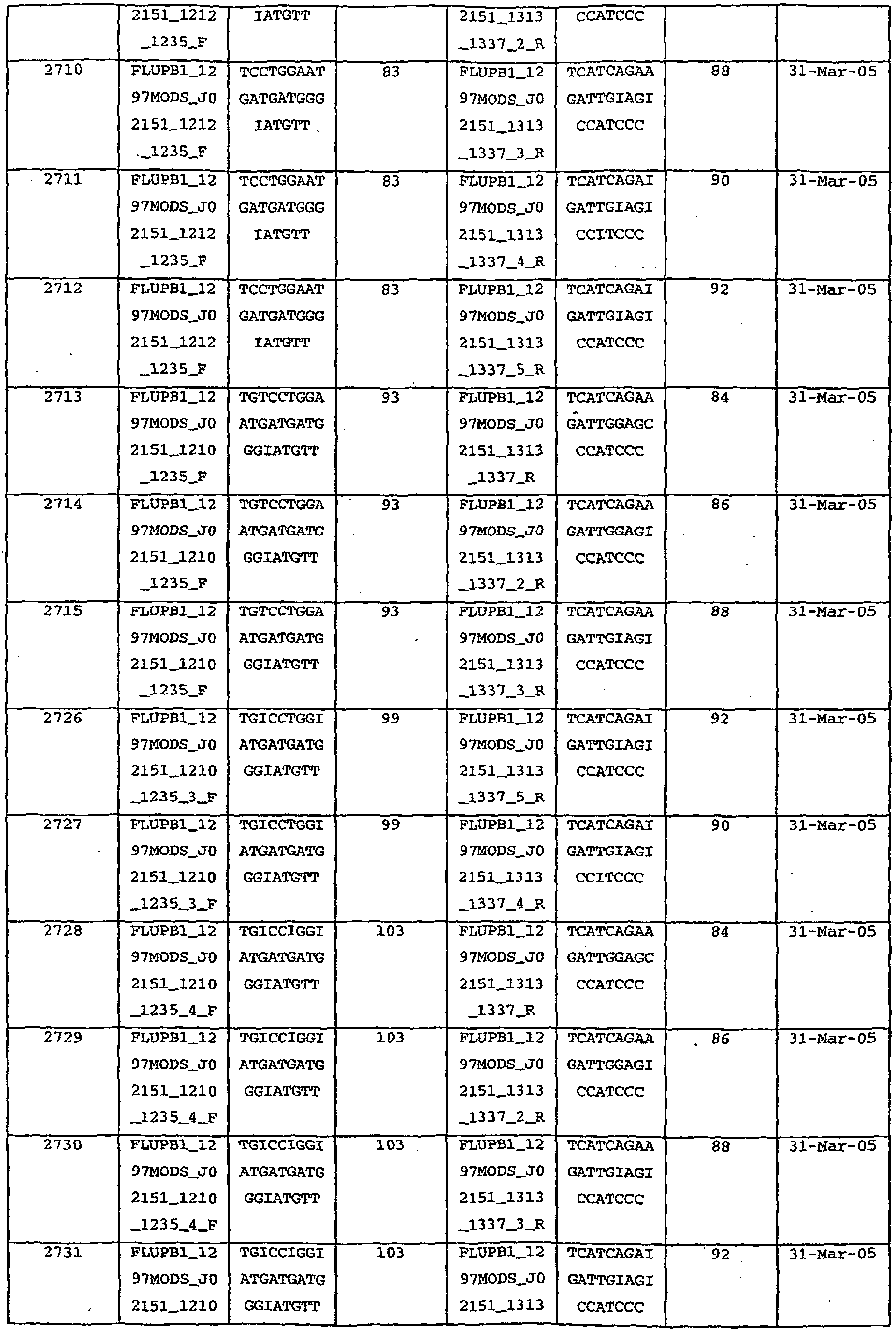

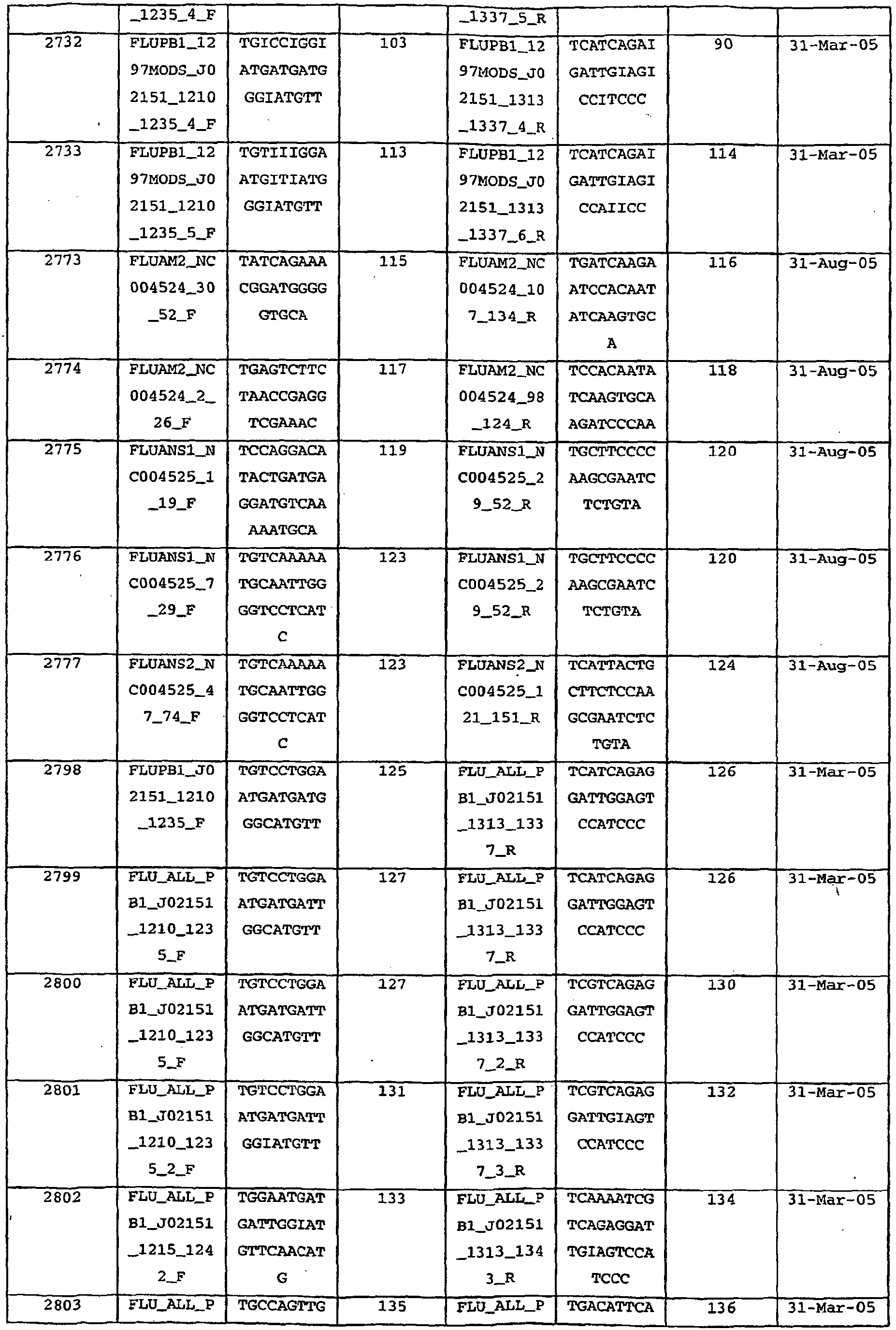

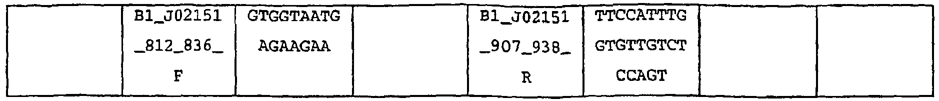

These primers were tested against a panel of influenza viral isolates obtained from ATCC (Manassas, VA 20108). A series of T7-tagged primers were designed to be specific to Influenza A and B species for each of the above viral genes. The purpose of this exercise was to generate *in vitro* transcripts (IVT) for one or more segments of the viral genes that could be used for primer validation and quantitation of the ATCC viral stock. The T7-primers targeted to PB1 and NUC segments of influenza A (VR-1520) and influenza B (VR-296) to generate four cDNA clones, labeled IVT-1520A-PB1, IVT-1520A-NP, IVT-296-PB1 and IVT-296-NP.

As shown in the gel photographs of Figure 3, the broad primers were able to detect both the influenza A and the influenza B constructs, at ∼100 copies input concentration. The more specific primers were sensitive to the stochastic limits of the PCR reaction (approximately 3-15 copies). Using these IVT constructs, a series of limiting dilution experiments were performed against the ATCC stocks (VIR1520 and VIR296) to estimate their genome concentrations. These estimates were used for validation of the rest of the primers described in Table 2. Finally, the primers were tested against the panel of test viruses listed above at 1000 and 100 genome copies. The test panel included 8 influenza A isolates (6 H1N1 isolates and 2 H3N2 isolates) and 7 influenza B isolates, all obtained from ATCC (Table 3). All influenza A and B primers worked against the corresponding test isolates at 1000 genome copies and several also worked with 100 genome copies. A subset of these primers were chosen for further consideration, based on bioinformatics analyses of their ability to differentiate the host for influenza A virus species and sub-types.

**Table 3: Strains of Influenza Virus Species A and B used for Testing Primer Pairs of Table 2**

| **ATCC Number** | **Influenza Virus Species** | **Strain Name** |
|---|---|---|
| VR-1520 | Influenza A virus | A/WS/33 (H1N1 [TC adapted]) |
| VR-1469 | Influenza A virus | A/PR/8/34, TC Adapted (H1N1) |
| VR-897 | Influenza A virus | A/New Jersey/8/76 (Hsw N1) (H1N1) |
| CR-546 | Influenza A1 virus | A1/Denver/1/57 (H1N1) |
| VR-96 | Influenza A virus | A/Weiss/43 (H1N1) |
| VR-95 | Influenza A virus | A/PR/8/34 (H1N1) |
| VR-544 | Influenza A virus | H3N2 A/Hong Kong/8/68 |
| VR-822 | Influenza A virus | A/Victoria/3/75 (H3N2) |
| VR-296 | Influenza B virus | B/Maryland/1/59 |
| VR-295 | Influenza B virus | B/Taiwan/2/62 |
| VR-101 | Influenza B virus | B/Lee/40 |
| VR-790 | Influenza B virus | B/Russia/69 |
| VR-789 | Influenza B virus | B/R75 |
| VR-1535 | Influenza B virus | B/Lee/40 TC Adapted |

Base compositions of the amplified products from several of the primer pairs were analyzed to determine a sub-set of primers that would provide rapid identification of influenza A viruses and would include strain resolution. Analysis using single primer pairs resulted in 50% resolution among the entire group. Further resolution was obtained using more than one primer pair for triangulation identification. A combination of four primer pairs yields >90% resolution at the species level. Further analysis of the data generated in triangulation identification provided grouping into various H and N types along with host species specificity with greater than 98% resolution. With the exception of a single Avian H5N1 species group (with 9 isolates) that was not resolved from 3 human H5N1 strains, all other avian isolates were clearly distinguished from each other and from other species types. The exception was from the 2004 outbreak isolates in Vietnam and Thailand. Human H5N1 viruses from this 2004 outbreak were homogeneous and similar to the avian H5N1 species (Cimons, ASM News (2005), 71 (9), 420-4). Similarly, two of the human H3N2 isolates from Japan in 1996/97 were indistinguishable at the host level on the basis of the four primer pairs described here from four Japanese swine isolates from the same time period. For these exceptions, the viruses were identified using the primer pairs and base composition analysis; however, the host species would have been assumed as avian or swine because the viruses were avian and swine viruses, respectively. Other data (not shown) indicates that this approach would clearly work for other host/type combinations as well.

### Example 2: Sample Preparation and PCR

Samples were processed to obtain viral genomic material using a Qiagen QIAamp Virus BioRobot MDx Kit (Valencia, CA 91355). Resulting genomic material was amplified using an MJ Thermocycler Dyad unit (BioRad laboratories, Inc., Hercules, CA 94547) and the amplicons were characterized on a Bruker Daltonics MicroTOF instrument (Billerica, MA 01821). The resulting molecular mass measurements were converted to base compositions and were queried into a database having base compositins indexed with primer pairs and bioagents.

All PCR reactions were assembled in 50 .micor.L reaction volumes in a 96-well microtiter plate format using a Packard MPII liquid handling robotic platform (Perkin Elmer, Bostan, MA 02118) and M.J. Dyad thermocyclers (BioRad, Inc., Hercules, CA 94547). The PCR reaction mixture consisted of 4 units of Amplitaq Gold, 1x buffer II (Applied Biosystems, Foster City, CA), 1.5 mM MgCl.sub.2, 0.4 M betaine, 800 .micro.M dNTP mixture and 250 nM of each primer. The following typical PCR conditions were used: 95.deg.C for 10 min followed by 8 cycles of 95.deg.C for 30 seconds, 48.deg.C for 30 seconds, and 72.deg.C 30 seconds with the 48.deg.C annealing temperature increasing 0.9.deg.C with each of the eight cycles. The PCR was then continued for 37 additional cycles of 95.deg.C for 15 seconds, 56.deg.C for 20 seconds, and 72.deg.C 20 seconds. Those ordinarily skilled in the art will understand PCR reactions.

### Example 3: Solution Capture Purification of PCR Products for Mass Spectrometry with Ion Exchange Resin-Magnetic Beads

For solution capture of nucleic acids with ion exchange resin linked to magnetic beads, 25 micor.1 of a 2.5 mg/mL suspension of BioClone amine terminated supraparamagnetic beads (San Diego, CA 92126) were added to 25 to 50 .micro.1 of a PCR (or RT-PCR) reaction containing approximately 10 pM of an amplicon. The above suspension was mixed for approximately 5 minutes by vortexing or pipetting, after which the liquid was removed after using a magnetic separator. The beads containing bound PCR amplicon were then washed three times with 50mM ammonium bicarbonate/50% MeOH or 100mM ammonium bicarbonate/50% MeOH, followed by three more washes with 50% MeOH. The bound PCR amplicon was eluted with a solution of 25mM piperidine, 25mM imidazole, 35% MeOH which included peptide calibration standards.

### Example 4: Mass Spectrometry and Base Composition Analysis

The ESI-FTICR mass spectrometer is based on a Bruker Daltonics (Billerica, MA) Apex II 70e electrospray ionization Fourier transform ion cyclotron resonance mass spectrometer that employs an actively shielded 7 Tesla superconducting magnet. The active shielding constrains the majority of the fringing magnetic field from the superconducting magnet to a relatively small volume. Thus, components that might be adversely affected by stray magnetic fields, such as CRT monitors, robotic components, and other electronics, can operate in close proximity to the FTICR spectrometer. All aspects of pulse sequence control and data acquisition were performed on a 600 MHz Pentium II data station running Bruker's Xmass software under Windows NT 4.0 operating system. Sample aliquots, typically 15 .micro.1, were extracted directly from 96-well microtiter plates using a CTC HTS PAL autosampler (LEAP Technologies, Carrboro, NC) triggered by the FTICR data station. Samples were injected directly into a 10 .micor.1 sample loop integrated with a fluidics handling system that supplies the 100 .micor.1 /hr flow rate to the ESI source. Ions were formed via electrospray ionization in a modified Analytica (Branford, CT) source employing an off axis, grounded electrospray probe positioned approximately 1.5 cm from the metalized terminus of a glass desolvation capillary. The atmospheric pressure end of the glass capillary was biased at 6000 V relative to the ESI needle during data acquisition. A counter-current flow of dry N.sub.2 was employed to assist in the desolvation process. Ions were accumulated in an external ion reservoir comprised of an rf-only hexapole, a skimmer cone, and an auxiliary gate electrode, prior to injection into the trapped ion cell where they were mass analyzed. Ionization duty cycles > 99% were achieved by simultaneously accumulating ions in the external ion reservoir during ion detection. Each detection event consisted of 1M data points digitized over 2.3 s. To improve the signal-to-noise ratio (S/N), 32 scans were co-added for a total data acquisition time of 74 s.

The ESI-TOF mass spectrometer is based on a Bruker Daltonics MicroTOF.sup.TM. Ions from the ESI source undergo orthogonal ion extraction and are focused in a reflectron prior to detection. The TOF and FTICR are equipped with the same automated sample handling and fluidics described above. Ions are formed in the standard MicroTOF.sup.TM ESI source that is equipped with the same off-axis sprayer and glass capillary as the FTICR ESI source. Consequently, source conditions were the same as those described above. External ion accumulation was also employed to improve ionization duty cycle during data acquisition. Each detection event on the TOF was comprised of 75,000 data points digitized over 75 .micro.s.

The sample delivery scheme allows sample aliquots to be rapidly injected into the electrospray source at high flow rate and subsequently be electrosprayed at a much lower flow rate for improved ESI sensitivity. Prior to injecting a sample, a bolus of buffer was injected at a high flow rate to rinse the transfer line and spray needle to avoid sample contamination/carryover. Following the rinse step, the autosampler injected the next sample and the flow rate was switched to low flow. Following a brief equilibration delay, data acquisition commenced. As spectra were co-added, the autosampler continued rinsing the syringe and picking up buffer to rinse the injector and sample transfer line. In general, two syringe rinses and one injector rinse were required to minimize sample carryover. During a routine screening protocol a new sample mixture was injected every 106 seconds. More recently a fast wash station for the syringe needle has been implemented which, when combined with shorter acquisition times, facilitates the acquisition of mass spectra at a rate of just under one spectrum/minute.

Raw mass spectra were post-calibrated with an internal mass standard and deconvoluted to monoisotopic molecular masses. Unambiguous base compositions were derived from the exact mass measurements of the complementary single-stranded oligonucleotides. Quantitative results are obtained by comparing the peak heights with an internal PCR calibration standard present in every PCR well at 500 molecules per well. Calibration methods are commonly owned and disclosed in U.S. Provisional Patent Application Serial No. 60/545,425.

### Example 5: De Novo Determination of Base Composition of Amplicons using Molecular Mass Modified Deoxynucleotide Triphosphates.

Because the molecular masses of the four natural nucleobases have a relatively narrow molecular mass range (A = 313.058, G = 329.052, C = 289.046, T = 304.046, values in Daltons-See Table 4), a persistent source of ambiguity in assignment of base composition can occur as follows: two nucleic acid strands having different base composition may have a difference of about 1 Da when the base composition difference between the two strands is G↔A (-15.994) combined with C ↔ T (+15.000). For example, one 99-mer nucleic acid strand having a base composition of A₂₇G₃₀C₂₁T₂₁ has a theoretical molecular mass of 30779.058 while another 99-mer nucleic acid strand having a base composition of A₂₆G₃₁C₂₂T₂₀ has a theoretical molecular mass of 30780.052 is a molecular mass difference of only 0.994 Da. A 1 Da difference in molecular mass may be within the experimental error of a molecular mass measurement and thus, the relatively narrow molecular mass range of the four natural nucleobases imposes an uncertainty factor in this type of situation. One method for removing this theoretical 1 Da uncertainty factor uses amplification of a nucleic acid with one mass-tagged nucleobase and three natural nucleobases.

Addition of significant mass to one of the 4 nucleobases (dNTPs) in an amplification reaction, or in the primers themselves, will result in a significant difference in mass of the resulting amplicon (greater than 1 Da) arising from ambiguities such as the G ↔ A combined with C ↔ T event (Table 4). Thus, the same the G ↔ A (-15.994) event combined with 5-Iodo-C ↔ T (-110.900) event would result in a molecular mass difference of 126.894 Da. The molecular mass of the base composition A₂₇G₃₀5-Iodo-C₂₁T₂₁ (33422.958) compared with A₂₆G₃₁5-Iodo-C₂₂T₂₀, (33549.852) provides a theoretical molecular mass difference is +126.894. The experimental error of a molecular mass measurement is not significant with regard to this molecular mass difference. Furthermore, the only base composition consistent with a measured molecular mass of the 99-mer nucleic acid is A₂₇G₃₀5-Iodo-C₂)T₂₁. In contrast, the analogous amplification without the mass tag has 18 possible base compositions.

**Table 4: Molecular Masses of Natural Nucleobases and the Mass-Modified Nucleobase 5-Iodo-C and Molecular Mass Differences Resulting from Transitions**

| **Nucleobase** | **Molecular Mass** | **Transition** | **Molecular Mass** |
|---|---|---|---|
| A | 313.058 | A-->T | -9.012 |
| A | 313.058 | A-->C | -24.012 |
| A | 313.058 | A-->5-Iodo-C | 101.888 |
| A | 313.058 | A-->G | 15.994 |
| T | 304.046 | T-->A | 9.012 |
| T | 304.046 | T-->C | -15.000 |
| T | 304.046 | T-->5-Iodo-C | 110.900 |
| T | 304.046 | T-->G | 25.006 |
| C | 289.046 | C-->A | 24.012 |
| C | 289.046 | C-->T | 15.000 |
| C | 289.046 | C-->G | 40.006 |
| 5-Iodo-C | 414.946 | 5-Iodo-C-->A | -101.888 |
| 5-Iodo-C | 414.946 | 5-Iodo-C-->T | -110.900 |
| 5-Iodo-C | 414.946 | 5-Iodo-C-->G | -85.894 |
| G | 329.052 | G-->A | -15.994 |
| G | 329.052 | G-->T | -25.006 |
| G | 329.052 | G-->C | -40.006 |
| G | 329.052 | G-->5-Iodo-C | 85.894 |

Mass spectra of bioagent-identifying amplicons can be analyzed using a maximum-likelihood processor, such as is widely used in radar signal processing. This processor first makes maximum likelihood estimates of the input to the mass spectrometer for each primer by running matched filters for each base composition aggregate on the input data. This includes the response to a calibrant for each primer.

The algorithm emphasizes performance predictions culminating in probability-of-detection versus probability-of-false-alarm plots for conditions involving complex backgrounds of naturally occurring organisms and environmental contaminants. Matched filters consist of *a priori* expectations of signal values given the set of primers used for each of the bioagents. A genomic sequence database is used to define the mass base count matched filters. The database contains the sequences of known bacterial bioagents and includes threat organisms as well as benign background organisms. The latter is used to estimate and subtract the spectral signature produced by the background organisms. A maximum likelihood detection of known background organisms is implemented using matched filters and a running-sum estimate of the noise covariance. Background signal strengths are estimated and used along with the matched filters to form signatures which are then subtracted. The maximum likelihood process is applied to this "cleaned up" data in a similar manner employing matched filters for the organisms and a running-sum estimate of the noise-covariance for the cleaned up data.

The amplitudes of all base compositions of bioagent-identifying amplicons for each primer are calibrated and a final maximum likelihood amplitude estimate per organism is made based upon the multiple single primer estimates. Models of all system noise are factored into this two-stage maximum likelihood calculation. The processor reports the number of molecules of each base composition contained in the spectra. The quantity of amplicon corresponding to the appropriate primer set is reported as well as the quantities of primers remaining upon completion of the amplification reaction.

Base count blurring can be carried out as follows. Electronic PCR can be conducted on nucleotide sequences of the desired bioagents to obtain the different expected base counts that could be obtained for each primer pair. See for example, Schuler, Genome Res. 7:541-50, 1997; or the e-PCR program available from National Center for Biotechnology Information (NCBI, NIH, Bethesda, MD 20894). One illustrative embodiment uses one or more spreadsheets from a workbook comprising a plurality of spreadsheets (e.g., Microsoft Excel). First in this example, there is a worksheet with a name similar to the workbook name; this worksheet contains the raw electronic PCR data. Second, there is a worksheet named "filtered bioagents base count" that contains bioagent name and base count; there is a separate record for each strain after removing sequences that are not identified with a genus and species and removing all sequences for bioagents with less than 10 strains. Third, there is a worksheet, "Sheet1" that contains the frequency of substitutions, insertions, or deletions for this primer pair. This data is generated by first creating a pivot table from the data in the "filtered bioagents base count" worksheet and then executing an Excel VBA macro. The macro creates a table of differences in base counts for bioagents of the same species, but different strains. One of ordinary skill in the art understands the additional pathways for obtaining similar table differences without undo experimentation.

Application of an exemplary script, involves the user defining a threshold that specifies the fraction of the strains that are represented by the reference set of base counts for each bioagent The reference set of base counts for each bioagent may contain as many different base counts as are needed to meet or exceed the threshold. The set of reference base counts is defined by taking the most abundant strain's base type composition and adding it to the reference set and then the next most abundant strain's base type composition is added until the threshold is met or exceeded. The current set of data was obtained using a threshold of 55%, which was obtained empirically.

For each base count not included in the reference base count set for that bioagent, the script then proceeds to determine the manner in which the current base count differs from each of the base counts in the reference set. This difference may be represented as a combination of substitutions, Si=Xi, and insertions, Ii=Yi, or deletions, Di=Zi. If there is more than one reference base count, then the reported difference is chosen using rules that aim to minimize the number of changes and, in instances with the same number of changes, minimize the number of insertions or deletions. Therefore, the primary rule is to identify the difference with the minimum sum (Xi+Yi), or (Xi+Zi), *e.g.,* one insertion rather than two substitutions. If there are two or more differences with the minimum sum, then the one that will be reported is the one that contains the most substitutions.

Differences between a base count and a reference composition are categorized as one, two, or more substitutions, one, two, or more insertions, one, two, or more deletions, and combinations of substitutions and insertions or deletions. The different classes of nucleobase changes and their probabilities of occurrence have been delineated in U.S. Patent Application Publication No. 2004209260 (U.S. Application Serial No. 10/418,514).

### Example 6: Influenza Virus Surveillance Panel.

The compositions and methods described herein are useful for screening a sample suspected of comprising one or more unknown bioagents to determine the identity of at least one of the bioagents. The identification of the at least one bioagent is accomplished by generating base composition signatures for portions of genes shared by two or more members of the *orthomyxovirdae* family. The base composition signatures are then compared to a plurality of base composition signatures that are indexed to primer pairs and bioagents. The plurality of base composition signatures in this collection is at least two, is more preferably at least 5, is more preferably still at least 14, is more preferably still at least 19, is more preferably still at least 25 and is more preferably still at least 35. The base composition signatures comprising this plurality identify at least one bioagent when that bioagent's measured and calculated base composition signature is queried against the plurality.

Pan-influenza virus PCR primer sets were developed that are capable of amplifying all three influenza virus species (A, B, and C) and subtypes (HxNy) from different animal hosts (human, avian, swine, etc.) and to distinguish their essential molecular features using base composition signatures. Additional primers were designed that broadly amplify all known members of a particular species, but do not cross-amplify members of different species (e.g., influenza A- and influenza B-specific primers). A surveillance panel of eight primers was selected comprising one pan-influenza primer pair (primer pair 2798 in Table 2), five influenza A-specific primer pairs (primer pairs 1266, 1279, 1287, 2775 and 2777 in Table 2), and two influenza B-specific primer pairs (primer pairs 1261 and 1275 in Table 2). Figure 5. To measure the breadth of coverage and resolution offered by this panel we tested 92 different influenza virus isolates, including 63 avian isolates, 18 human influenza A isolates (eight H1N1, 10 H3N2), 6 human influenza B isolates, 4 swine isolates (including one novel type) and 1 equine isolate. The avian isolates were obtained from 16 different avian species sampled across North America and Asia/Middle East, representing 28 different H/N types and 29 HPAI (H5N1). Despite the diversity of this sample set, the broad-range primers generated amplicons from all isolates, while the base composition signatures from amplicons obtained with these primers distinguished the isolates as shown in figures 6a and 6b. Most isolates showed base compositions consistent with expected signatures for the corresponding H/N sub-types based on bioinformatic analysis of existing sequence data. Two of the isolates, however, showed previously unrepresented base composition signatures across several primer loci suggesting these might be novel influenza virus serotypes, and are noted as "New" serotypes in Figures 6a.

Base composition signatures further provide a multidimensional fingerprint of the genomes from various viruses. These fingerprints are visualized using three-dimensional plots as shown for three primer pairs in Figure 7. Each axis on the plot comprises base composition signatures for the notated gene using primer pairs 2798, 1266 and 1287 from Table 2. Data represented in the plot includes both bioinformatic analysis of sequence data from GenBank (hollow shapes) and experimental measurements from of base composition signatures from figures 6a and 6b (filled shape). In this example, human H3N2 and H1N1 viruses cluster independently and are separated by base composition differences on all three axes. Avian H5N1 and H1N1 are also in independent clusters and are separated along the axes by base composition. Additional resolution is provided by using additional primer pairs. The cubes (human isolates) nested in the spheres (avian isolates) in the avian H5N1 cluster (arrows) represent the recently reported instances of avian H5N1 virus isolated from humans.

A deeper level analysis of the clusters allows for surveillance (broad range priming to indicate whether or not influenza is present), speciation of the host (e.g., avian, human) and of the virus (e.g., influenza A, influenza B), sub-typing (e.g. H.sub.xN.sub.y) and genotyping (e.g., H.sub.5N.sub.1 - Egypt type, Iraq type). Figure 12 illustrates this point. Here the Avian H.sub.5N.sub.1 cluster from figure 7 is analyzed to determine further determine the genotype of the assayed viruses. Figure 7 indicated that the bioagents were influenza, were from humans and avian host species and were influenza A species. The base composition analysis in figure 7 further indicated that for the avian influenza A virus species, that there were two sub-types: H.sub.1N.sub.1 and H.sub.5N.sub.1. In figure 12, the analysis is taken deeper for the avian H.sub.5N.sub.1 subtype. In figure 12 it is seen that the various isolates are clustered together, with the exception of a single Egypt isolate that clusters closer with the Iraq isolates. Spatial differences within a cluster are slight variations in genotype.

### Example 7: Analysis of Human Clinical Sample Using the Current Compositions and Methods and Comparison with Traditional Clinical Diagnostic Methods.

Primer pairs and the PCR-ESI/MS method were used to analyze 656 blinded human clinical samples. The samples were collected during the seven year period from 1999 to 2006. Primer pairs 2798, 1266,1279, 1287, 2775, 2777, 1261 and 1275 were used for triangulation identification. Collection techniques included throat swabs, nasal swabs, and washes. The obtained results were compared with conventional analysis of the same samples by virus isolation and standard RT-PCR methods. Two hundred fifty-three samples were positive for influenza both by PCR-ESI/MS and by conventional culture/RT-PCR. Ten samples were positive by PCR-ESI/MS and negative by culture or RT-PCR and eight samples were positive by culture/RT-PCR and negative by PCR-ESI/MS, corresponding to 98% sensitivity and specificity for the PCR-ESI/MS method compared with conventional diagnostic methods.

Of the 253 influenza-positive samples, PCR-ESI/MS analysis identified 186 as influenza A virus and 67 as influenza B virus. Analysis of the base compositions of regions of the PB1, NP, M1, PA, NS1, and NS2 genes, coupled with a comparison with the predicted base compositions from published influenza virus sequences allowed inference of the H and N subtypes of these viruses with a high degree of accuracy. The human influenza A viruses were categorized into 152 H3N2 and 34 H1N1 subtypes based on conventional serological analysis and direct H and N typing by RT-PCR. Of course, inferences of the H and N types based upon association with other genes could be erroneous when the H or N gene segments re-assort and associate with a new set of other gene segments. Three of the H1N2 samples could not be sub-typed by the PCR-ES1/MS approach because these viral subtypes arose from re-assortment of the H gene from an H1N1 virus with other gene segments from H3N2 virus (Holmes, E.C., et al., PLoS Biol. (2005), 3, e300).

### Example 8: Identification of a Mixed Viral Population using the Current Compounds and Methods.

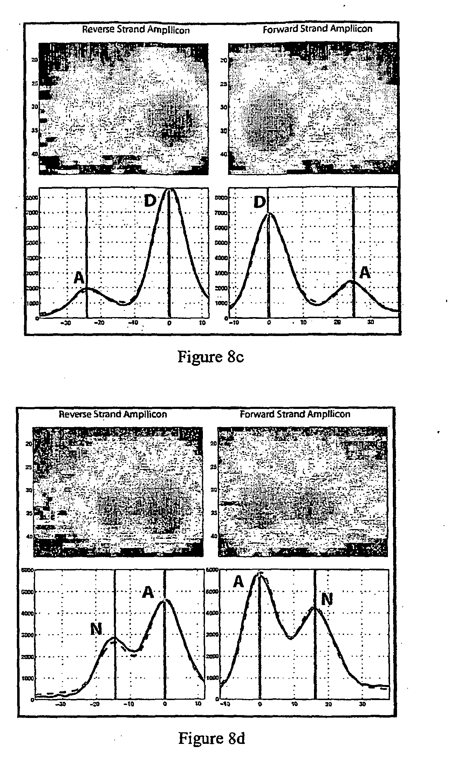

Co-infections with different influenza viruses can be identified with high sensitivity by PCR-ESI/MS because amplified viral nucleic acids having different base compositions appear in different positions in the mass spectrum. The dynamic range for mixed PCR-ESI/MS detections has previously been determined to be approximately 100:1 (Hofstadler, S. A. et al., Inter. J. Mass Spectrom. (2005) 242, 23), which allows for detection of viral variants with as low as 1% abundance in a mixed population. This detection using PCR-ESI/MS surveillance does not require secondary testing. Mixed influenza virus populations were identified in both original patient samples and cultures derived from them (Figure 8). Figure 8a shows the spectrum obtained from amplification of influenza A virus present in infected culture fluid with a primer pair that amplifies a region of M1 (primer pair 1279). The forward strand is on the left and reverse strand is to the right. The two viral species seen here differ from each other by an A to G single nucleotide polymorphism (SNP) within the targeted sequence and are present roughly as a 60:40 mixture in the sample. Figure 8b shows a spectrum from a patient sample using primer pair 2775 (NS1) and the lower abundance species is present at approximately 2% of the viral population. The forward strand shows a shoulder on the left side of the peak and the reverse strand shows a similar shoulder on the right side, corresponding respectively to an A to G variation and the complementary T to C mass shifts at approximately 2% of the amplitude of the main peaks. To demonstrate that these peaks represent mixed viral populations, the PCR amplicons were cloned and 450 independent colonies were sequenced. Only nine of these 450 clones had the A to G mutations, which correlated well with the measured amplitude of the low abundance peaks. Figure 8c and d show spectra from two additional clinical samples analyzed using primer pairs 1279 and 2775, respectively, each a mixture of two different circulating viruses observed in samples collected in the year 2006, containing the two viral species roughly as 20-40% mixtures. The genotypic characterization of these viruses is described in the example below.

### Example 9: Genotyping and Tracking of Influenza Virus.

Base composition signatures allow for both temporal and geographical tracking of outbreak strains. For instance, as shown in Figures 9a and 9b, analysis of PCR-ESI/MS base-composition signatures of the H3N2 positive isolates from patient samples showed excellent consistency with the known phylogenies of circulating viruses within the sampling period (*4*).

To understand the distribution of base compositions as related to influenza virus evolution, we analyzed both the complete genome sequences of world-wide human influenza virus (H3N2) sequences from Genbank and the base composition signatures generated using primer pairs 2798, 1266, 1279, 1287, 2775 and 2777. The Genbank sequences were collapsed into base composition signatures across the 6 loci used in this study (∼ 5% of virus genome). Each unique base composition signature at each target locus was assigned a unique letter code (e.g. A, B, C,), and the six-loci were concatenated together to form unique allele types (e.g. AADFAA). Experimentally measured base compositions shown in Figure 11 were also assigned a letter code. These base compositions were mapped back to the phylogenetic distribution of human H3N2 described by Holmes *et al.,* (Figure 11). Thus, 95 unique base composition types were determined from a total of 731 H3N2 base compositions obtained computationally from sequence data (shown on black) or experimentally measured (shown in red). In cases where the experimentally determined base compositions were consistent with sequenced isolates from Genbank, data are shown only for the sequenced strains. The tree was further extended with ongoing analysis of >900 influenza samples (200 positive for H3N2) collected during 2005-2006 from Texas and Baltimore.

Analysis of relatedness of these isolates by base compositions shows a distribution that is very similar to the sequence derived clades of Holmes *et al.* There is clear evidence for the presence of two different circulating clades up to the years 2002-03 and 2003-04. While the branch terminating in clade A represents the dominant branch between years 1999-2004, there is clear evidence for the co-circulation of the clade B branches in the same time period, worldwide. Strikingly, however, clade B isolates are the predominant influenza A viruses circulating world-wide post 2003-4 season. This is evidenced by analysis of sequence data in Genbank and experimentally determined base compositions from samples in recent years. The seasonal and geographical relatedness of the circulating viruses is indicated by the vertical bar and shows global evolutions of influenza virus across the various influenza virus seasons. For instance, analysis of recent samples from North America (Texas/Baltimore; 2005-6) showed the presence of a parent circulating virus type (genotype AADFAA), which was one of the circulating genotypes from the previous season from New Zealand (A/Christchurch/2005). Most of the other genotypes observed in this sample set are clearly one or two mutations away from this major genotype, although there is possible evidence for at least two other direct descendants from the previous seasons. All of the mutations observed in these isolates have been verified by direct sequencing of the PCR products and confirm the experimentally determined base compositions (data not shown). Thus, the base composition analysis described here could serve as a rapid tool for characterizing global spread and monitoring the emergence of novel influenza viruses.

### Example 10: High Throughput Influenza Detection and Analysis.

In an effective surveillance program, samples must be assayed rapidly. This has been accomplished using the current compositions and methods. To measure the throughput of the PCR-ESI/MS method, 336 respiratory specimens were analyzed using four of the primer sets. The experiment was designed to simulate sample analysis in a real-time surveillance laboratory, with nucleic acid extraction on over 300 samples completed during the work day by laboratory technicians(s) and PCR and mass spectrometry analysis proceeding in an automated fashion beginning in the afternoon, running throughout the night (unattended), and completed early the next morning. The 336 samples were analyzed in 26 hours (for a throughput of 312 samples per 24 hour period). The timetable for the high-throughput experiment is shown in Figure 10. An identical experiment retesting the same 336 samples was repeated the next day, providing evidence that this pace can be maintained for a weekly (5 day) throughput of more than 1,500 samples.

Various modifications to the description herein will be apparent to those skilled in the art from the foregoing description. Such modifications fall within the scope of the current invention and appended claims.

## Claims

1. An oligonucleotide primer pair wherein each primer member of the primer pair is independently up to 35 nucleobases in length and wherein the forward primer comprises SEQ ID NO: 125 and the reverse primer comprises SEQ ID NO: 126.

2. A kit comprising the oligonucleotide primer pair of claim 1.

3. The kit of claim 2 further comprising a plurality of primer pairs wherein one or more of the primer pairs are designed to target the NUC gene of influenza A virus, one or more of the primer pairs are designed to target the M1 gene of influenza A, one or more of the primer pairs are designed to target the PA gene of influenza A, one or more of the primer pairs are designed to target the NSI gene of influenza A, one or more of the primer pairs are designed to target the NS2 gene of influenza A, one or more of the primer pairs are designed to target the PB2 gene of influenza B, and one or more of the primer pairs are designed to target the NUC gene of influenza B, and wherein the plurality of primer pairs will generate amplicons that identify the genus, species, sub-species and genotype of one or more bioagent from the *orthomyxoviridae* family.

4. The kit of claim 3 wherein the one or more of the primer pairs designed to target the NUC gene of influenza A virus comprises SEQ ID NOS: 15:16, the one or more primer pairs designed to target the M1 gene of influenza A comprises SEQ ID NOS: 39:40, the one or more primer pairs designed to target the PA gene of influenza A comprises SEQ ID NOS: 55:56, the one or more primer pairs designed to target the NSI gene of influenza A comprises SEQ ID NOS: 119:120, the one or more primer pairs designed to target the NS2 gene of influenza A comprises SEQ ID NOS: 123:124, the one or more primer pairs designed to target the PB2 gene of influenza B comprises SEQ ID NOS: 31:32.

5. The kit of claim 3 further comprising one or more primer pairs wherein said one or more primer pairs is of a length of up to 35 nucleobases and has 100% sequence identity with any member from the group of primer pairs represented by SEQ ID NOs: 39:40, 15:16, 55:56, 5:6, 31:32, 123:124, 119:120, 1:2, 3:4, 7:8, 9:10, 11:12, 13:14, 17:18, 19:20, 21:22, 23:24, 25:26, 27:28, 29:30, 33:34, 35:36, 37:38, 41:42, 43:44, 45:46. 47:48, 49:50, 51:52, 53:54, 59:60, 61:62, 63:64, 65:66, 67:68, 69:70, 71:72, 73:74, 75:76, 125:78, 79:80, 81:82, 83:84, 83:86, 83:88, 83:90, 83:92, 93:84, 93:86, 93:88, 99:92, 99:90, 103:84, 103:86, 103:88, 103:92, 103:90, 113:114, 115:116, 117:118, 123:120, 123:124, 127:126, 127:130, 131:132, 133:134, 135:136.

6. A method for identification of an influenza virus comprising:
amplifying, in a sample suspected of comprising at least one bioagent, nucleic acid from said influenza virus using the composition of claim 1 to obtain an amplicon;
determining the molecular mass of said amplicon using a mass spectrometer;
calculating the base composition of said amplicon from said molecular mass; and
comparing said molecular mass or base composition with a plurality of molecular masses or base compositions indexed to known influenza virus bioagents and primer pairs, wherein a match between said molecular mass or base composition and a member of said plurality of molecular masses or base compositions identifies said unknown influenza virus.

7. A method of determining the presence or absence of an influenza virus in a sample comprising:
amplifying, in a sample suspected of comprising at least one bioagent, nucleic acid from said sample using the composition of claim 1 to obtain an amplicon;
determining the molecular mass of said amplicon using a mass spectrometer;
calculating the base composition of said amplicon from said molecular mass; and
comparing said molecular mass or base composition of said amplicon with a collection of molecular masses or base compositions indexed to a known bioagent and to a primer pair, wherein a match between the measured molecular mass or calculated base composition and one of the molecular mass or base composition in the collection indicates the presence of influenza virus in said sample.

8. A combination of at least three primer pairs for identifying the genus, species, subtype and genotype of at least two bioagents belonging to the *orthomyxovirdae* family, wherein the primer pairs individually bind to one of a gene sequence comprising PB1, NUC, M1, PA, NS1, NS2, and PB2 and thereby generate corresponding amplicons from each of the two or more bioagents, the amplicons being compatible with a mass spectrometer for generating a molecular mass measurement and for calculating a base composition signature that corresponds to each of the two or more bioagents, and wherein the combination comprises a primer pair comprising a forward primer that has 100% sequence identity with SEQ ID NO: 125, and a reverse primer that has 100% sequence identity with SEQ ID NO: 126.

9. The combination of claim 8 wherein the primer pairs individually bind to one of a gene sequence comprising PB1, PA, and NS1, and wherein the combination comprises a primer pair comprising a forward primer that has 100% sequence identity with SEQ ID NO: 125, and a reverse primer that has 100% sequence identity with SEQ ID NO: 126.

10. The combination of claim 9 wherein the primer members of a first primer pair comprise a forward primer that has 100% sequence identity with SEQ ID NO: 125, and a reverse primer that has 100% sequence identity with SEQ ID NO: 126; the primer members of a second primer pair comprise a forward primer that has 100% sequence identity with SEQ ID NO: 55, and a reverse primer that has 100% sequence identity with SEQ ID NO: 56; and the primer members of a third primer pair comprise a forward primer that has 100% sequence identity with SEQ ID NO: 119, and a reverse primer that has 100% sequence identity with SEQ ID NO: 120.

11. A method for generating a base composition signature to identify at least one member of the orthomyxovirdae family comprising the steps of:
(a) obtaining a primer pair according to claim 1;
(b) contacting the primer pair to a sample suspected of comprising a unknown bioagent;
(c) generating an amplicon;
(d) obtaining a mass measurement of the amplicon using a mass spectrometer; and
(e) calculating a base composition signature from the molecular mass data, wherein the base composition signature is queried against a database of base composition signatures indexed to the corresponding primer pairs and the bioagent identity.

## Patentansprüche

1. Oligonukleotidprimerpaar, wobei jedes Primerelement des Primerpaars unabhängig bis zu 35 Nukleobasen lang ist und wobei der Vorwärts-Primer die SEQ ID NO: 125 umfasst und der reverse Primer die SEQ ID NO: 126 umfasst.

2. Kit, umfassend das Oligonukleotidprimerpaar nach Anspruch 1.

3. Kit nach Anspruch 2, das ferner eine Mehrzahl von Primerpaaren umfasst, wobei eines oder mehrere der Primerpaare derart gestaltet ist/sind, dass es/sie das NUC-Gen des Influenza A-Virus ansteuer(t/n), eines oder mehrere der Primerpaare derart gestaltet ist/sind, dass es/sie das M1-Gen von Influenza A ansteuer(t/n), eines oder mehrere der Primerpaare derart gestaltet ist/sind, dass es/sie das PA-Gen von Influenza A ansteuer(t/n), eines oder mehrere der Primerpaare derart gestaltet ist/sind, dass es/sie das NS1-Gen von Influenza A ansteuer(t/n), eines oder mehrere der Primerpaare derart gestaltet ist/sind, dass es/sie das NS2-Gen von Influenza A ansteuer(t/n), eines oder mehrere der Primerpaare derart gestaltet ist/sind, dass es/sie das PB2-Gen von Influenza B ansteuer(t/n), eines oder mehrere der Primerpaare derart gestaltet ist/sind, dass es/sie das NUC-Gen von Influenza B ansteuer(t/n), und wobei die Mehrzahl an Primerpaaren zu Amplikons führen, die Gattung, Art, Unterart und Genotyp von einem Bioagens oder mehreren Bioagentien aus der Familie *Orthomyxovirdae* identifizieren.

4. Kit nach Anspruch 3, wobei das eine oder die mehreren der Primerpaare, das/die derart gestaltet ist/sind, dass es/sie das NUC-Gen des Influenza A-Virus ansteuer(t/n), die SEQ ID NO: 15:16 umfasst, das eine oder die mehreren Primerpaare, das/die derart gestaltet ist/sind, dass es/sie das M1-Gen von Influenza A ansteuer(t/n), die SEQ ID NO: 39:40 umfasst, das eine oder die mehreren Primerpaare, das/die derart gestaltet ist/sind, dass es/sie das PA-Gen von Influenza A ansteuer(t/n), die SEQ ID NO: 55:56 umfasst, das eine oder die mehreren Primerpaare, das/die derart gestaltet ist/sind, dass es/sie das NS1-Gen von Influenza A ansteuer(t/n), die SEQ ID NO: 119:120 umfasst, das eine oder die mehreren Primerpaare, das/die derart gestaltet ist/sind, dass es/sie das NS2-Gen von Influenza A ansteuer(t/n), die SEQ ID NO: 123:124 umfasst, das eine oder die mehreren Primerpaare, das/die derart gestaltet ist/sind, dass es/sie das PB2-Gen von Influenza B ansteuer(t/n), die SEQ ID NO: 31:32 umfasst.

5. Kit nach Anspruch 3, das ferner ein oder mehrere Primerpaare umfasst, wobei das eine oder die mehreren Primerpaare bis zu 35 Nukleobasen lang sind und 100% Sequenzidentität mit einem beliebigen Mitglied aus der Gruppe von Primerpaaren besitzen, die durch SEQ ID NO: 39:40, 15:16, 55:56, 5:6, 31:32, 123:124, 119:120, 1:2, 3:4, 7:8, 9:10, 11:12, 13:14, 17:18, 19:20, 21:22, 23:24, 25:26, 27:28, 29:30, 33:34, 35:36, 37:38, 41:42, 43:44, 45:46, 47:48, 49:50, 51:52, 53:54, 59:60, 61:62, 63:64, 65:66, 67:68, 69:70, 71:72, 73:74, 75:76, 125:78, 79:80, 81:82, 83:84, 83:86, 83:88, 83:90, 83:92, 93:84, 93:86, 93:88, 99:92, 99:90, 103:84, 103:86, 103:88, 103:92, 103:90, 113:114, 115:116, 117:118, 123:120, 123:124, 127:126, 127:130, 131:132, 133:134, 135:136 dargestellt werden.

6. Verfahren zum Identifizieren eines Influenza-Virus, umfassend:
Amplifizieren, in einer Probe, von der vermutet wird, dass sie mindestens ein Bioagens umfasst, von Nukleinsäure von dem Influenza-Virus unter Verwendung der Zusammensetzung nach Anspruch 1, um ein Amplikon zu erhalten,
Bestimmen der Molekülmasse des Amplikons unter Verwendung eines Massenspektrometers,
gegebenenfalls Berechnen der Basenzusammensetzung des Amplikons aus der Molekülmasse und
Vergleichen der Molekülmasse oder der Basenzusammensetzung mit einer Mehrzahl an Molekülmassen oder Basenzusammensetzungen, die mit bekannten Influenzavirus-Bioagentien und -Primerpaaren gekoppelt sind, wobei eine Übereinstimmung zwischen der Molekülmasse oder der Basenzusammensetzung und einem Mitglied der Mehrzahl von Molekülmassen oder Basenzusammensetzungen das unbekannte Influenza-Virus identifiziert.

7. Verfahren zum Bestimmen der Anwesenheit oder Abwesenheit eines Influenza-Virus in einer Probe, umfassend:
Amplifizieren, in einer Probe, von der vermutet wird, dass sie mindestens ein Bioagens umfasst, von Nukleinsäure aus der Probe unter Verwendung der Zusammensetzung nach Anspruch 1, um ein Amplikon zu erhalten,
Bestimmen der Molekülmasse des Amplikons unter Verwendung eines Massenspektrometers,
Berechnen der Basenzusammensetzung des Amplikons aus der Molekülmasse und
Vergleichen der Molekülmasse oder der Basenzusammensetzung des Amplikons mit einer Zusammenstellung von Molekülmassen oder Basenzusammensetzungen, die mit einem bekannten Bioagens und einem Primerpaar gekoppelt sind, wobei eine Übereinstimmung zwischen der gemessenen Molekülmasse oder der berechneten Basenzusammensetzung und einer der Molekülmassen oder Basenzusammensetzungen in der Zusammenstellung auf das Vorliegen von Influenza-Virus in der Probe hindeutet.

8. Kombination von mindestens drei Primerpaaren zum Identifizieren von Gattung, Art, Unterart und Genotyp von mindestens zwei Bioagentien, die zur Familie *Orthomyxovirdae* gehören, wobei die Primerpaare einzeln an eine aus einer Gensequenz, umfassend PB1, NUC, M1, PA, NS1, NS2 und PB2, binden und dadurch zu entsprechenden Amplikons von jedem der zwei oder mehreren Bioagentien führen, wobei die Amplikons kompatibel sind mit einem Massenspektrometer zum Erzeugen einer Molekülmassenmessung und zum Berechnen einer Basenzusammensetzungssignatur, die jeweils den zwei oder mehreren Bioagentien entspricht, und wobei die Kombination ein Primerpaar umfasst, das einen Vorwärts-Primer mit 100% Sequenzidentität zu SEQ ID NO: 125 und einen reversen Primer mit 100% Sequenzidentität zu SEQ ID NO: 126 umfasst.

9. Kombination nach Anspruch 8, wobei die Primerpaare einzeln an eine aus einer Gensequenz, umfassend PB1, PA und NS1, binden und wobei die Kombination ein Primerpaar umfasst, das einen Vorwärts-Primer mit 100% Sequenzidentität zu SEQ ID NO: 125 und einen reversen Primer mit 100% Sequenzidentität zu SEQ ID NO: 126 umfasst.

10. Kombination nach Anspruch 9, wobei die Primerelemente eines ersten Primerpaars einen Vorwärts-Primer mit 100% Sequenzidentität zu SEQ ID NO: 125 und einen reversen Primer mit 100% Sequenzidentität zu SEQ ID NO: 126 umfassen, die Primerelemente eines zweiten Primerpaars einen Vorwärts-Primer mit 100% Sequenzidentität zu SEQ ID NO: 55 und einen reversen Primer mit 100% Sequenzidentität zu SEQ ID NO: 56 umfassen und die Primerelemente eines dritten Primerpaars einen Vorwärts-Primer mit 100% Sequenzidentität zu SEQ ID NO: 119 und einen reversen Primer mit 100% Sequenzidentität zu SEQ ID NO: 120 umfassen.

11. Verfahren zum Erstellen einer Basenzusammensetzungssignatur zum Identifizieren mindestens eines Mitglieds der Familie *Orthomyxovirdae,* umfassend die folgenden Schritte:
(a) Erhalten eines Primerpaars nach Anspruch 1,
(b) Zusammenbringen der Primerpaars mit einer Probe, von der vermutet wird, dass sie ein unbekanntes Bioagens umfasst,
(c) Herstellen eines Amplikons,
(d) Erhalten einer Massenmessung des Amplikons unter Verwendung eines Massenspektrometers,
(e) Berechnen einer Basenzusammensetzungssignatur aus den Molekülmassedaten, wobei die Basenzusammensetzungssignatur gegen eine Datenbank von Basenzusammensetzungssignaturen, die mit den entsprechenden Primerpaaren und der Bioagens-Identität gekoppelt sind, abgeglichen wird.

## Revendications

1. Paire d'amorces oligonucléotidiques dans laquelle chaque membre d'amorce de la paire d'amorces est indépendamment de jusqu'à 35 nucléobases de longueur et dans laquelle l'amorce sens comprend SEQ ID NO: 125 et l'amorce antisens comprend SEQ ID NO: 126.

2. Kit comprenant la paire d'amorces oligonucléotidiques de la revendication 1.

3. Kit de la revendication 2 comprenant en outre une pluralité de paires d'amorces dans lequel une ou plusieurs des paires d'amorces sont conçues pour cibler le gène NUC du virus de la grippe A, une ou plusieurs des paires d'amorces sont conçues pour cibler le gène M1 de la grippe A, une ou plusieurs des paires d'amorces sont conçues pour cibler le gène PA de la grippe A, une ou plusieurs des paires d'amorces sont conçues pour cibler le gène NSI de la grippe A, une ou plusieurs des paires d'amorces sont conçues pour cibler le gène NS2 de la grippe A, une ou plusieurs des paires d'amorces sont conçues pour cibler le gène PB2 de la grippe B, et une ou plusieurs des paires d'amorces sont conçues pour cibler le gène NUC de la grippe B, et la pluralité de paires d'amorces générant des amplicons qui identifient le genre, l'espèce, la sous-espèce et le génotype d'un ou plusieurs agents biologiques de la famille *orthomyxoviridae.*

4. Kit de la revendication 3 dans lequel les une ou plusieurs paires d'amorces conçues pour cibler le gène NUC du virus de la grippe A comprennent SEQ ID NO: 15:16, les une ou plusieurs paires d'amorces conçues pour cibler le gène M1 de la grippe A comprennent SEQ ID NO: 39:40, les une ou plusieurs paires d'amorces conçues pour cibler le gène PA de la grippe A comprennent SEQ ID NO: 55:56, les une ou plusieurs paires d'amorces conçues pour cibler le gène NSI de la grippe A comprennent SEQ ID NO: 119:120, les une ou plusieurs paires d'amorces conçues pour cibler le gène NS2 de la grippe A comprennent SEQ ID NO: 123:124, les une ou plusieurs paires d'amorces conçues pour cibler le gène PB2 de la grippe B comprennent SEQ ID NO: 31:32.

5. Kit de la revendication 3 comprenant en outre un ou plusieurs paires d'amorces dans lequel lesdites une ou plusieurs paires d'amorces a une longueur de jusqu'à 35 nucléobases et a 100 % d'identité de séquence avec un membre quelconque du groupe de paires d'amorces représenté par SEQ ID NO: 39:40, 15:16, 55:56, 5:6, 31:32, 123:124, 119:120, 1:2, 3:4, 7:8, 9:10, 11:12, 13:14, 17:18, 19:20, 21:22, 23:24, 25:26, 27:28, 29:30, 33:34, 35:36, 37:38, 41:42, 43:44, 45:46, 47:48, 49:50, 51:52, 53:54, 59:60, 61:62, 63:64, 65:66, 67:68, 69:70, 71:72, 73:74, 75:76, 125:78, 79:80, 81:82, 83:84, 83:86, 83:88, 83:90, 83:92, 93:84, 93:86, 93:88, 99:92, 99:90, 103:84, 103:86, 103:88, 103:92, 103:90, 113:114, 115:116, 117:118, 123:120, 123:124, 127:126, 127:130, 131:132, 133:134, 135:136.

6. Procédé pour l'identification d'un virus de la grippe comprenant :
l'amplification, dans un échantillon suspecté de comprendre au moins un agent biologique, d'acide nucléique dudit virus de la grippe en utilisant la composition de la revendication 1 pour obtenir un amplicon ;
la détermination de la masse moléculaire dudit amplicon en utilisant un spectromètre de masse ;
le calcul de la composition de base dudit amplicon à partir de ladite masse moléculaire ; et
la comparaison de ladite masse moléculaire ou de la composition de base à une pluralité de masses moléculaires ou compositions de base indexées à des agents biologiques et des paires d'amorces de virus de la grippe connu, où une correspondance entre ladite masse moléculaire ou composition de base et un membre de ladite pluralité de masses moléculaires ou compositions de base identifie ledit virus de la grippe connu.

7. Procédé de détermination de la présence ou l'absence d'un virus de la grippe dans un échantillon comprenant :
l'amplification, dans un échantillon suspecté de comprendre au moins un agent biologique, d'acide nucléique dudit échantillon en utilisant la composition de la revendication 1 pour obtenir un amplicon ;
la détermination de la masse moléculaire dudit amplicon en utilisant un spectromètre de masse ;
le calcul de la composition de base dudit amplicon à partir de ladite masse moléculaire ; et
la comparaison de ladite masse moléculaire ou composition de base dudit amplicon à une collection de masses moléculaires ou compositions de base indexées à un agent biologique et à une paire d'amorce connus, où une correspondance entre la masse moléculaire mesurée ou la composition de base calculée et une des masses moléculaires ou compositions de base dans la collection indique la présence du virus de la grippe dans ledit échantillon.

8. Combinaison d'au moins trois paires d'amorces pour identifier le genre, l'espèce, la sous-espèce et le génotype d'un ou plusieurs agents biologiques de la famille *orthomyxoviridae,* dans laquelle les paires d'amorces se lient individuellement à l'une de séquences de gène comprenant PB1, NUC, M1, PA, NS1, NS2, et PB2 et génèrent ainsi les amplicons correspondants de chacun des deux bioagents ou plus, les amplicons étant compatibles avec un spectromètre de masse pour générer une mesure de masse moléculaire et pour calculer une signature de composition de base qui correspond à chacun des deux bioagents ou plus, et où la combinaison comprend une paire d'amorces comprenant une amorce sens qui a 100 % d'identité de séquence avec SEQ ID NO: 125 et une amorce antisens qui a 100 % d'identité de séquence avec SEQ ID NO: 126.

9. Combinaison de la revendication 8 dans laquelle les paires d'amorces se lient individuellement à l'une de séquences de gène comprenant PB1, PA, et NS1, et dans laquelle la combinaison comprend une paire d'amorces comprenant une amorce sens qui a 100 % d'identité de séquence avec SEQ ID NO: 125, et une amorce inverse qui a 100 % d'identité de séquence avec SEQ ID NO: 126.

10. Combinaison de la revendication 9 dans laquelle les membres d'amorce d'une première paire d'amorces comprend une amorce sens qui a 100 % d'identité de séquence avec SEQ ID NO: 125, et une amorce inverse qui a 100 % d'identité de séquence avec SEQ ID NO: 126 ; les membres d'amorce d'une deuxième paire d'amorces comprend une amorce sens qui a 100 % d'identité de séquence avec SEQ ID NO: 55, et une amorce inverse qui a 100 % d'identité de séquence avec SEQ ID NO: 56 ; et les membres d'amorce d'une troisième paire d'amorces comprennent une amorce sens qui a 100 % d'identité de séquence avec SEQ ID NO: 119, et une amorce antisens qui a 100 % d'identité de séquence avec SEQ ID NO: 120.

11. Procédé pour générer une signature de composition de base pour identifier au moins un membre de la famille *orthomyxoviridae* comprenant les étapes de :
(a) obtention d'une paire d'amorces selon la revendication 1 ;
(b) mise en contact de la paire d'amorces avec un échantillon suspecté de comprendre un agent biologique inconnu ;
(c) génération d'un amplicon ;
(d) obtention d'une mesure de masse de l'amplicon en utilisant un spectromètre de masse ; et
(e) calcul d'une signature de composition de base à partir des données de masse moléculaire, où la signature de composition de base est recherchée dans une base de données de signatures de composition de base indexée aux paires d'amorces correspondantes et l'identité d'agent biologique.
